# EUROPÄISCHE PATENTANMELDUNG

(11) **EP 1 531 007 A2**
(43) Veröffentlichungstag der Anmeldung: **18.05.2005**
(21) Anmeldenummer: 04105802.5
(22) Anmeldetag: 16.11.2004
(51) Int. Cl.: B05B 11/00

(54) **Spender mit kosmetischen Zubereitungen, die Hilfsmittel zur Gängighaltung des Spenders enthalten**

(30) Priorität: 17.11.2003 DE 10354051
(71) Anmelder: Beiersdorf AG, 20245 Hamburg (DE)
(72) Erfinder: Lanzendoerfer, Ghita, 22087, Hamburg (DE); Riedel, Heidi, 22529, Hamburg (DE); Ruppert, Stephan, 20259, Hamburg (DE); Eckers, Lorenz, 21255, Tostedt (DE); Mundt, Claudia, 28207, Bremen (DE); Kallmayer, Volker, 22525, Hamburg (DE)

(57) **Zusammenfassung**

Kosmetisches Produkt umfassend einen Spender für pastöse Produkte mit einem das pastöse Produkt enthaltenden, im Wesentlichen zylindrischen Behälter (1), der bodenseitig einen unter dem Druck der Außenatmosphäre an einer Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, dadurch gekennzeichnet dass die Fördereinrichtung ein zu dem Behälter (1) und dem Kopfstück (3) längsverschiebliches Förderelement (5) umfasst, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlassöffnung (53) und eine Förderkanalauslassöffnung (58) aufweist, die durch eine Verschiebebewegung des Förderelementes (5) relativ zu dem Kopfstück (3) in eine Stellung bringbar ist, in der sich die Förderkanalauslassöffnung (58) zu dem Ausgabekanal (32) öffnet, sowie eine wasserhaltige kosmetisch und/oder dermatologische Zubereitung, die ein Hilfsmittel zur Gängighaltung des Spenders gewählt aus der Gruppe der Polyole mit 2 bis 6 Kohlenstoffatomen und 2 bis 6 Hydroxy- oder Alkoxygruppen und/oder der Tenside, die die Oberflächenspannung des Produktes auf <30mN/m herabsetzen.

## Beschreibung

Die vorliegende Erfindung betrifft eine kosmetische Zubereitung, einen Spender für kosmetische Zubereitungen mit einem die kosmetische Zubereitung enthaltenden, im wesentlichen zylindrischen Behälter, der bodenseitig einen unter dem Druck der Außenatmosphäre an einer Behälterinnenwand gleitverschieblichen Nachlaufkolben aufweist und an seinem oberen Ende ein zu dem Behälter gleitverschiebliches Kopfstück trägt, welches einen mit dem Behälter kommunizierend verbindbaren Ausgabekanal für das Produkt aufweist und auf eine handbetätigbare Fördereinrichtung mit einer volumenveränderlichen Förderkammer für das Produkt einwirkt.

Spender mit gleitverschieblichen Nachlaufkolben und handbetätigbare Fördereinrichtung mit einer volumenveränderlichen Förderkammer sind als ambulante Vorratsbehälter in einer Vielzahl von Anwendungsbeispielen bekannt, z. B. für die Körperpflege, in der Medizin für die Applikation von Medikamenten oder auch bei der Bereitstellung pastöser Lebensmittel im Handel. Entsprechend vielgestaltig ist auch die Ausgestaltung der für die Bereitstellung der sehr unterschiedlichen pastösen Massen verwendeten Spender, vor allen in Bezug auf ihren unmittelbaren Förder-und Handhabungsmechanismus.

Ein solcher Spender ist beispielsweise aus der EP-A-0 230 252 bekannt. Bei diesem vorbekannten Spender weist die handbetätigbare Fördereinrichtung einen Förderkolben auf, durch welchen das Volumen der Förderkammer veränderbar ist. Der Förderkolben ist mit einem Rohrstück verrastet, welches einstückig an dem Kopfteil ausgeformt ist. Bei der Benutzung des Spenders wird das Kopfstück von einer Ausgangslage durch Handbetätigung in axialer Richtung in Richtung auf den Behälter verschoben.

Diese Verschiebebewegung führt direkt zu einer Gleitbewegung des Förderkolbens entlang der Innenwandung der Förderkammer unter Verringerung des Volumens derselben. Der hierbei aufgebaute Innendruck in der Förderkammer führt zunächst zur Öffnung eines in dem Förderkolben ausgebildeten ellipsenförmig eine Durchlassöffnung des Förderkolbens abdeckenden Rückschlagventils, durch welches dann das pastöse Produkt bei einer weiteren Verringerung des Volumens der Förderkammer in Richtung auf den Ausgabekanal zur Entnahme an einer Produktabgabeöffnung, die an dem Kopfteil ausgebildet ist, gefördert wird.

Bei dem vorbekannten Spender muss in der Förderkammer zunächst ein die Verschlusskräfte des Rückschlagventils überwindender Innendruck aufgebaut werden.

Auch das Fördern der pastösen Masse durch das Rückschlagventil führt zu einem Druckverlust, welcher insofern nachteilig ist, als dass zur Kompensation dieses Druckverlustes erhöhte Druckkräfte aufgewendet werden müssen, um das Kopfteil in Richtung auf den Behälter axial zu verschieben. Darüber hinaus besteht das Problem, dass durch das Rückschlagventil gefördertes Produkt in denjenigen Förderbereichen des Spenders ansteht, die dem Rückschlagventil in Förderrichtung nachgeordnet sind. Diese Förderbereiche kommunizieren jedoch ständig über die Produktabgabeöffnung mit der Umgebung, was zu einer Beeinträchtigung der pastösen Produkte führen kann. Lebensmittel werden häufig hinsichtlich ihrer geschmacklichen und farblichen Qualität durch Oxidation beeinflusst. Arzneimittel können unter Lufteinfluss an ihrer Wirksamkeit beeinträchtigt werden.
Die Schrift WO 03/004374 offenbart einen Spender der eingangs genannten Art, der dahingehend verbessert ist, dass eine Betätigung mit geringeren Betätigungskräften möglich ist und darüber hinaus eine Beeinträchtigung des abzugebenden pastösen Produktes durch Oxidation vermindert wird. Dabei umfasst die Fördereinrichtung ein zu dem Behälter und dem Kopfstück längsverschiebliches Förderelement, welches einen in der Förderkammer gleitverschieblichen Förderkolben aufweist, der mit einem Förderschaft verbunden ist, welcher einen Förderkanal umfänglich umgibt, der eine mit der Förderkammer kommunizierende Förderkanaleinlassöffnung und eine Förderkanalauslassöffnung aufweist, die durch eine Verschiebebewegung des Förderelementes relativ zu dem Kopfstück in eine Stellung bringbar ist, in der sich die Förderkanalauslassöffnung zu dem Ausgabekanal öffnet.

Bei dem schriftgemäßen Spender öffnet sich die Förderkammer zu dem Ausgabekanal über eine Förderkanalauslassöffnung, die über eine Längsverschiebung des Förderelementes relativ zu dem Kopfstück freigegeben wird. Diese Relativbewegung wird vorzugsweise dadurch erzielt, dass das Kopfstück handbetätigt wird, d. h. in axialer Richtung in Richtung auf den Behälter gleitverschoben wird. Der Durchlass des pastösen Produktes von der Förderkammer zu der Produktabgabeöffnung am Ende des Ausgabekanals wird dementsprechend bereits durch eine translatorische Bewegung des Kopfstückes relativ zu dem Förderelement freigegeben. Ein vorheriger Druckaufbau in der Förderkammer, wie er beim gattungsbildenden Stand der Technik zur Freigabe des Durchganges erforderlich war, ist nicht erforderlich. Dementsprechend werden die Betätigungskräfte zur Abgabe von pastösen Produkten aus dem Spender verringert.

Bei dem schriftgemäßen Spender ist nachgeordnet zu der Förderkammer ein Förderkanal vorgesehen, der von einem Förderschaft umgeben ist. Am Ende dieses Förderkanals wird das aus der Förderkammer ausgeförderte pastöse Produkt durch die Förderkanalauslassöffnung in den Ausgabekanal abgegeben. Erst nach Abgabe des Produktes aus der Förderkanalauslassöffnung steht dieses in dem Ausgabekanal an.

Der verbleibende Ausgabekanal ist jedenfalls kürzer als bei den üblicherweise verwendeten Spendern. Dementsprechend wird deutlich weniger Volumen pastöser Masse durch eventuelle Oxidationsvorgänge beeinträchtigt. Die verbleibende Restlänge des Ausgabekanals kann insbesondere bei solchen Produkten, die gegenüber Oxidation hochgradig anfällig sind, dadurch verkürzt werden, dass der Ausgabekanal sich in Verlängerung der Stirnseite des Kopfstückes nach außen öffnet.

Bei einer vorteilhaften Ausgestaltung des schriftgemäßen Spenders wird die Förderkanalauslassöffnung an der Umfangsfläche des Förderschaftes ausgespart und an dem Kopfstück eine die Förderkanalauslassöffnung in der Ausgangsstellung der Fördereinrichtung abdeckende Buchse vorgesehen, so dass sich bei einer Hubbewegung des Kopfteiles zum Ausfördern pastöser Masse auf einfache Weise eine Freigabe der Förderkanalauslassöffnung dadurch ergibt, dass der Förderschaft relativ zu der Buchse bewegt wird. Diese bevorzugte Ausgestaltung ist nicht nur einfach, sondern erlaubt auch eine Anordnung der Förderkanalauslassöffnung in unmittelbarer Nähe der Einlassöffnung des Ausgabekanals für das zu fördernde Produkt.

Mit Rücksicht auf eine gute axiale Führung der Fördereinrichtung relativ zu dem Kopfstück wird die vorerwähnte Buchse vorzugsweise als Führungsbuchse für die Fördereinrichtung ausgebildet und hat wenigstens eine mit der Umfangsfläche des Förderschaftes zusammenwirkende Führungsfläche.

Im Hinblick auf einen zwangsläufigen Verschluss der Förderkanalauslassöffnung bei Rückstellung des Kopfstückes in die Ausgangslage wird gemäß einer bevorzugten Weiterbildung der vorliegenden Erfindung vorgeschlagen, dass an dem Kopfteil und an der Fördereinrichtung Mitnehmermittel vorgesehen sind, durch welche die Fördereinrichtung nach Handbetätigung bei Rückstellung des Kopfteiles in die Ausgangsstellung mitgenommen wird.

Die vorerwähnten Mitnehmermittel sind auf einfache Weise vorzugsweise durch eine an der Buchse ausgebildete Mitnehmerschulter gebildet, die mit einem an dem Förderschaft angeformten Mitnehmerkranz zusammenwirkt. Dieser Mitnehmerkranz ist vorzugsweise endseitig an dem Förderschaft angeformt, so dass die unterhalb des Mitnehmerkranzes ausgesparte Förderkanalauslassöffnung in der Ausgangsstellung durch Anlage des Mitnehmerkranzes an Wandungen des Kopfteiles abgedichtet werden kann.

Bei der vorerwähnten bevorzugten Ausgestaltung kann das in dem Ausgabekanal anstehende Volumen dadurch weiter verringert werden, dass die Mitnehmerschulter endseitig an der Buchse und am Übergang zu dem Ausgabekanal und der Mitnehmerkranz im stirnseitigen Endbereich des endseitig verschlossenen Förderschaftes ausgebildet ist, wie dies gemäß einer bevorzugten Weiterbildung der vorliegenden Erfindung vorgeschlagen wird. Bei dieser bevorzugten Ausgestaltung deckt die endseitig an dem Förderschaft angeordnete Schaftkappe den Ausgabekanal in der Ausgangsstellung der Fördereinrichtung im wesentlichen bündig ab und weist vorzugsweise den Mitnehmerkranz auf.

Gemäß einer bevorzugten Weiterbildung der vorliegenden Erfindung erfolgt die Betätigung des Förderkolbens vorzugsweise über die endseitigen Stirnflächen der Führungsbuchse. Bei dieser bevorzugten Weiterbildung überragt der Förderkolben den Förderschaft radial zur Ausbildung einer ringförmigen Anlagefläche für eine Druckfläche, die stirnseitig an der Führungsbuchse ausgebildet ist und die in der Ausgangsstellung mit axialem Abstand zu der Anlagefläche angeordnet und durch axiales Verschieben des Kopfstückes in Richtung auf den Behälter an die Anlagefläche anlegbar ist.

Ebenfalls mit Rücksicht auf eine konstruktive Vereinfachung wird gemäß einer weiteren bevorzugten Ausgestaltung der vorliegenden Erfindung vorgeschlagen, die Innenwandung der Förderkammer durch eine Innenhülse auszubilden, welche an der kopfstückseitigen Stirnseite des Behälters vorgesehen ist. Dabei überragt die Innenhülse die Stirnseite des Behälters an der dem Kopfstück zugewandten Seite. Vorzugsweise ist die Innenhülse zur Verringerung der Bauteile einstückig an dem Behälter angeformt.

Zur einfachen Zentrierung des Kopfstückes bei der Montage des Spenders und leichten Befestigung des Kopfstückes an dem Behälter wird gemäß einer bevorzugten Weiterbildung der vorliegenden Erfindung ein Kopfgegenstück vorgeschlagen, das einen topfförmig auf die vorerwähnte Innenhülse gestülpten Haltezylinder sowie einen konzentrisch zu dem Haltezylinder angeordneten, die Gleitverschiebung des Kopfstückes führenden Führungszylinder aufweist. Der Führungszylinder und/oder der Haltezylinder erlauben eine leichte konzentrische Ausrichtung des Kopfteiles zu dem Zylinder. Ferner verbessert der Führungszylinder die Führung der Hubbewegung des Kopfstückes bei der Betätigung des Spenders.

Bei einer weiteren bevorzugten Ausgestaltung des schriftgemäßen Spenders, bei der das förderkammerseitige Ende des Führungszylinders einen Förderkolbenanschlag für den Förderkolben ausbildet, wird zum Einen eine relativ längliche Führung für den Förderkolben und zum Anderen auf einfache Weise eine Hubbegrenzung des Förderkolbens bereitgestellt. Eine derartige Hubbegrenzung hält beispielsweise das Kopfteil in der Ausgangslage an dem Behälter fest, wenn die Mitnehmermittel in Wirkverbindung stehen.

Vorzugsweise weist der Haltezylinder eine bodenseitige Ringschulter auf, welche eine Anlagefläche für eine Schraubenfeder ausbildet, die das Kopfstück in der Ausgangsstellung unter Vorspannung hält. Dies bietet den Vorteil, dass die äußere Umfangsfläche des Haltezylinders die Schraubenfeder innenseitig umgibt und somit ein Knicken der Feder verhindert. Die Ringschulter ist bei dieser bevorzugten Ausgestaltung auf die Stirnseite des Behälters aufgesetzt und ist somit insbesondere geeignet, das Kopfgegenstück in axialer Richtung gegenüber dem Behälter festzulegen.

Gemäß einer weiteren, besonders bevorzugten Ausgestaltung sind das Kopfgegenstück und das Kopfstück als vorgefertigte Spenderkomponente ausgebildet. Hierbei sind besonders bevorzugt das Kopfstück und das Kopfgegenstück mit ihrer äußeren Mantelfläche jeweils topfförmig übereinander geschoben, wobei das Kopfgegenstück wenigstens einen Anschlag zur Begrenzung der axialen Verschiebebewegung des Kopfstücks relativ zu dem Kopfgegenstück aufweist. In dem von den Mantelflächen umgebenden Innenraum befindet sich bei einer derartigen Ausgestaltung vorzugsweise ein Rückstellelement, beispielsweise die vorstehend erwähnte Schraubenfeder, welche das Kopfstück und das Kopfgegenstück in axialer Richtung beabstandet unter Vorspannung hält. Der vorerwähnte Anschlag grenzt die axiale Verschiebebewegung des Kopfstücks, d. h. sorgt nach dem Zusammenbau von Kopfstück und Kopfgegenstück unter Einschluss der Feder für den Zusammenhalt der beiden gegeneinander verschieblichen Bauteile. Die derart gebildete Spenderkomponente kann auf unterschiedlich ausgestaltete Behälter aufgesetzt werden, was eine wirtschaftliche Herstellung des Spenders für sehr unterschiedliche Anwendungen und Behältervolumina erlaubt.

Eine besonders einfache und haltbare Verbindung zwischen der vorgefertigten Spenderkomponente und dem Behälter wird dadurch gebildet, dass die Spenderkomponente mit dem Behälter über an dem Kopfgegenstück und der Stirnseite des Behälters ausgebildete Rastmittel verrastet sind.

Vorzugsweise ist bei dem schriftgemäßen Spender das Kopfstück derart längenverschieblich, dass das Kopfstück mittels Handbetätigung von der Ausgangsstellung zunächst um eine erste axiale Wegstrecke zur Anlage an den Förderkolben bei gleichzeitiger Freilegung der Förderkanalauslassöffnung in dem Ausgabekanal in eine Mittelposition bringbar ist und das Kopfteil danach bei fortschreitender axialer Verschiebung unter Mitnahme des Förderkolbens von der Mittelposition in eine Ausgabe-Endposition bringbar ist, in welcher die Förderkammer durch Verschiebung des Förderkolbens ihr kleinstes Volumen erreicht hat. Bei dieser bevorzugten Ausgestaltung erfolgt das Freilegen der Förderkanalauslassöffnung und das Komprimieren der Substanz in dem Förderkanal im Rahmen einer gleichgerichteten Bewegung des Kopfstücks in Richtung auf den Behälter. Diese bevorzugte Ausgestaltung erlaubt eine konstruktiv einfache Lösung des schriftgemäßen Spenders, bei welcher das Kopfstück unmittelbar auf den Förderkolben wirkt und diesen nach Freilegung der Förderkanalauslassöffnung zum Fördern pastöser Masse antreibt. Diese Bewegung des Kopfstücks erfolgt üblicherweise gegen die Kraft eines Vorspannelementes, beispielsweise einer Feder, die dafür Sorge trägt, dass bei einer Entlastung des Kopfstücks dieses von der Ausgabe-Endposition weg von dem Behälter drückt. Bei dieser Bewegung wird zuerst die axiale Wegstrecke a zurückgelegt, d. h. die Förderkanalauslassöffnung wird wieder verschlossen. Bei dieser Verschließbewegung findet eine Relativbewegung zwischen dem Förderschaft und dem Ausgabekanal statt, bei welcher das Volumen des Ausgabekanals an dessen Einlass vergrößert wird. Dadurch wird die in dem Ausgabekanal befindliche pastöse Masse in Richtung auf die Pumpkammer zurückgezogen, also von der Produktabgabeöffnung des Ausgabekanals in dem Kopfteil entfernt.

Gemäß einer bevorzugten Ausgestaltung der Erfindung befindet sich an dieser Produktabgabeöffnung ein Verschließteil. Das Verschließteil ist vorzugsweise derart beschaffen, dass es sich aufgrund einer Druckdifferenz zwischen dem Ausgabekanal und der Atmosphäre zur Abgabe des pastösen Produktes öffnet. Wird - wie vorstehend erwähnt - die pastöse Masse in dem Ausgabekanal weg von der Produktabgabeöffnung zurückgezogen, so führt dies zu einem relativen Unterdruck in dem Ausgabekanal, welcher dafür sorgt, dass das Verschließteil die Produktabgabeöffnung besonders wirkungsvoll abdichtet.

Im Hinblick auf eine möglichst gute Abdichtung ist es zu bevorzugen, die Produktabgabeöffnung um einen in dem Ausgabekanal angeordneten Verschlussdorn auszubilden.

Dieser Verschlussdorn ist vorzugsweise einstückig an dem Kopfteil ausgeformt. Das ebenfalls ringförmig ausgebildete Verschlussteil weist eine an dem Verschlussdorn dichtend anlegbare Dichtlippe auf, welche bei einem wirksamen Unterdruck den Ausgabekanal wirkungsvoll verschließt, jedoch beim Ausfördern des pastösen Produktes eine verhältnismäßig große Produktabgabeöffnung freigibt, durch welche bei relativ geringem Druckverlust das Produkt ausgefördert werden kann.

Besonders wirtschaftlich lässt sich ein Verschließteil hoher Wirksamkeit mittels Zweikomponenten-Spritzgießen an dem Kopfteil ausformen, wie dies gemäß einer bevor zugten Weiterbildung der vorliegenden Erfindung vorgeschlagen wird. Bei dieser Ausgestaltung ist das Verschließteil fest mit dem Kopfteil verbunden. Vorzugsweise ist das Verschließteil aus einem weichelastischen Kunststoff, besonders bevorzugt aus einem thermoplastischen Elastomer gebildet. Es hat sich gezeigt, dass insbesondere durch einen thermoplastischen Elastomer eine wirkungsvolle Abdichtung der Produktabgabeöffnung erreicht werden kann.

Es hat sich gezeigt, dass das Material für das Dichtteil sich besonders bevorzugt zur Ausbildung einer Funktionsfläche an der stirnseitigen Außenfläche des Kopfteiles genutzt werden kann. Eine derartige Funktionsfläche kann beispielsweise eine die haptischen Eigenschaften verbessernde Drückerfläche sein, gegen welche der Benutzer des Spenders bei dessen Benutzung drückt. Eine derartige Funktionsfläche wird vorzugsweise durch einen Überzug zumindest stirnseitig an der Außenseite des Kopfteiles ausgebildet. Das Verschließteil sowie der Überzug sind einstückig ausgeformt, vorzugsweise mittels Zweikomponenten-Spritzgießen nach der spritzgießtechnischen Herstellung des Kopfteiles.

Weitere Einzelheiten, Vorteile und Merkmale der vorliegenden Erfindung ergeben sich aus der nachfolgenden Beschreibung eines Ausführungsbeispiels in Verbindung mit der Zeichnung. In dieser zeigen :
Figur 1 eine Längsschnittansicht eines ersten Ausführungsbeispieles eines schriftgemäßen Spenders ;
Figur 2 eine Längsschnittansicht eines zweiten Ausführungsbeispiels des schriftgemäßen Spenders.

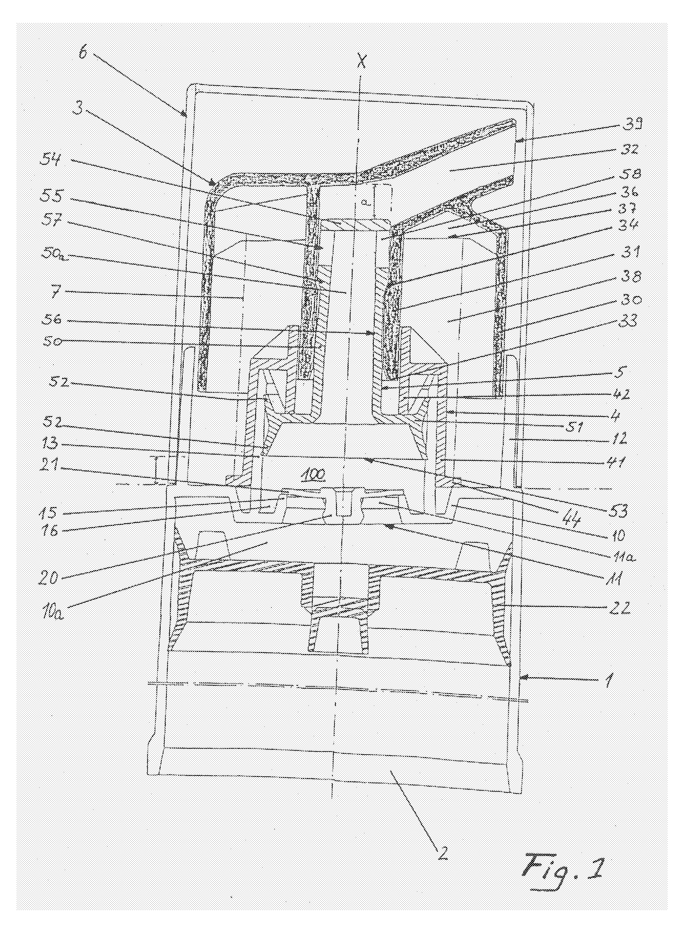

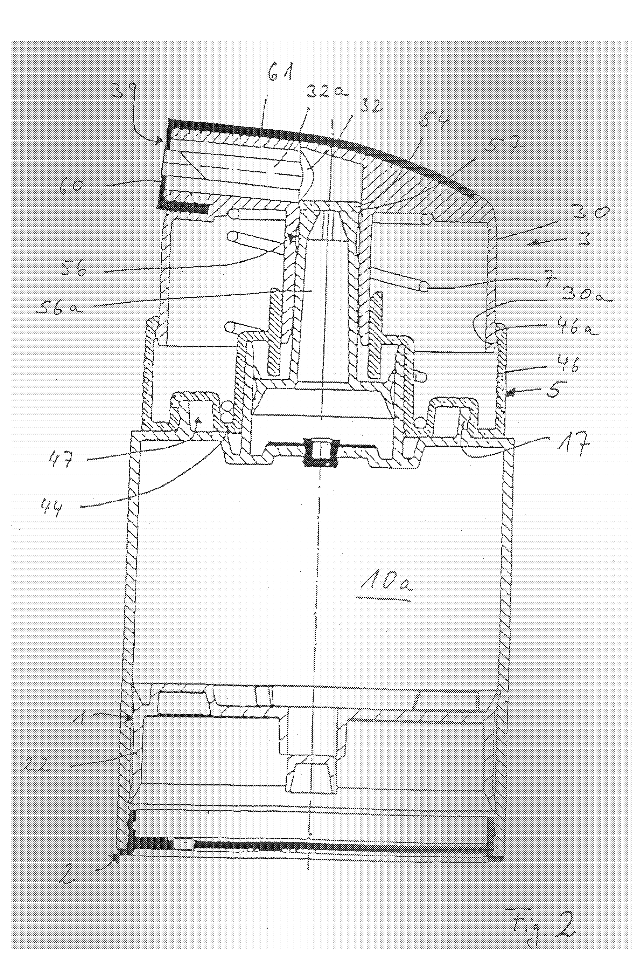

Das in Figur 1 gezeigte Ausführungsbeispiel eines schriftgemäßen Spenders hat einen Behälter 1, der topfförmig ausgebildet ist und an seiner Unterseite mit einer Bodenplatte 2 verbunden ist, welche mit dem Behälter 1 verrastet ist. An seiner anderen Stirnseite weist der Behälter 1 eine kopfseitige Abdeckung 10 auf, in der eine Behälteröffnung 11 ausgespart ist. Diese Abdeckung 10 ist auf der dem Behälter 1 abgewandten Seite zur Aufnahme eines Spenderkopfes bestehend aus einem Kopfstück 3, einem Kopfgegenstück 4 und einem Druckkolben 5 ausgebildet. Der Spender weist ferner eine auf eine sich oberhalb der Abdeckung 10 erstreckende Außenhülse 12 des Behälters 1 aufgeschobene Verschlusskappe 6 auf. Der Behälter 1, die Bodenplatte 2, das Kopfgegenstück 4 sowie der Druckkolben 5 sind als rotationssymmetrische Bauteile ausgebildet und zu einer Mittellängssachse X konzentrisch angeordnet. Zwischen dem Kopfstück 3 und dem Kopfgegenstück 4 befindet sich eine schematisch angedeutete Schraubenfeder 7, durch welche das Kopfstück 3 in der in Figur 1 gezeigten Ausgangsstellung gegenüber dem Kopfgegenstück 4 vorgespannt gehalten ist.

Das Kopfstück 3 weist einen zylindrischen Außenmantel 30 auf, welcher radial innerhalb unmittelbar benachbart zu der Außenhülse 12 des Behälters 1 und konzentrisch zu dieser angeordnet ist. Die Außenhülse 12 des Behälters 1 überragt das behälterseitige Ende des Außenmantels 30 in axialer Richtung. Dementsprechend erscheint das in Figur 1 gezeigte Ausführungsbeispiel des Spenders auch bei abgenommener Verschlusskappe als geschlossene Einheit bestehend aus dem Behälter 1 und dem Kopfstück 3. Wie nachfolgend näher erläutert wird, ist das Kopfstück 3 sowie der Druckkolben 5 längstverschieblich gegenüber dem Behälter 1 gehalten, wobei der Druckkolben 5 darüber hinaus längstverschieblich gegenüber dem Kopfstück 3 ist.

Die zylindrische Wandung des Behälters 1 umschließt einen Innenraum 10a zur Aufnahme der kosmetischen oder dermatologischen Zubereitung. In der Behälteröffnung 11 erstrecken sich sternförmig ausgerichtete Haltestege 11 a. Auf der dem Innenraum 10a abgewandten Seite der Abdeckung 10 ist konzentrisch zu der Behälteröffnung 11 eine zylindrische Innenhülse 13 angeordnet, die in axialer Richtung von der Außenhülse 12 überragt wird und die eine Förderkammer 100 umgibt. Die Innenwandung der Innenhülse 13 ist glatt. Der Boden der Förderkammer 100 wird durch die Abdeckung 10 des Behälters 1 gebildet. Die Abdeckung 10 weist einen in die Förderkammer 100 hineinragenden Ringkranz 15 auf, welcher die Behälteröffnung 11 umgibt und zwischen sich und der Innenhülse 13 einen Ringspalt 16 bildet.

Der Druckkolben 5 weist einen im wesentlichen zylindrischen, innen hohlen Förderschaft 50 auf, an dessen einem Ende ein Förderkolben 51 einstückig angeformt ist. Der Förderkolben 51 überragt den Förderschaft 50 radial und hat an seiner äußeren Umfangsfläche jeweils obere und untere Dichtlippen 52, die den im wesentlichen ringförmig ausgebildeten Förderkolben 51 in axialer Richtung überragen. Auf einer dem Förderschaft 50 zugewandten Stirnseite bildet der Förderkolben 51 eine ringförmige Anlagefläche aus.

Der Förderschaft 50 weist an seinem einen Ende eine in der Mitte des ringförmigen Förderkolbens 51 ausgesparte Förderkanaleinlassöffnung 53 auf. An seinem anderen Ende ist der Förderschaft 50 stirnseitig durch eine Schaftkappe 54 verschlossen. Die Schaftkappe 54 deckt einen Zylinderabschnitt 55 des Förderschaftes 50 ab, der gegenüber dem übrigen Schaftbereich 56 im Durchmesser vergrößert ist. Zwischen diesem Schaftbereich 56 und dem Zylinderabschnitt 55 befindet sich ein schräg nach außen geneigter Mitnehmerkranz 57. Zwischen dem Mitnehmerkranz 57 und der Schaftkappe 54 sind an der äußeren Umfangsfläche des Zylinderabschnitts 55 mehrere Förderkanalauslassöffnungen 58 verteilt ausgespart. Zwischen den Förderkanalauslassöffnungen 58 erstrecken sich in Umfangsrichtung Haltestege, welche die Schaftkappe 54 tragen. Die Förderkanaleinlassöffnung 53 kommuniziert über einen von dem Förderschaft 50 umgebenden Förderkanal 50a mit den Förderkanalauslassöffnungen 58 und bildet eine Förderpassage für die pastöse Substanz, die frei von Rückschlagventilen ist.

Das Kopfstück 3 weist einen, zylindrischen Außenmantel 30 auf, zu dem konzentrisch eine innen hohle Führungsbuchse 31 angeordnet ist, die mit einem Ausgabekanal 32 kommuniziert. Das stirnseitige Ende der Führungsbuchse 32 bildet eine stirnseitige Druckfläche 33 aus, welche in axialer Richtung von dem Außenmantel 30 überragt wird. Die Führungsbuchse 31 weist benachbart zu der stirnseitigen Druckfläche 33 einen ersten Buchsenabschnitt auf, der einen geringeren Innendurchmesser hat, als der in aus Förderrichtung der pastösen Substanz dahinter liegender zweiter Buchsenabschnitt. Zwischen dem ersten und dem zweiten Buchsenabschnitt ist eine Mitnehmerschulter 34 ausgebildet, welche die beiden Abschnitte unterschiedlicher Buchsendurchmesser über eine Schräge miteinander verbindet. Der zweite Buchsenabschnitt mündet in einen in den von der Mittellängsachse X seitlich abgehenden Ausgabekanal 32.

In etwa rechtwinkliger Erstreckung zu der Mittellängsachse X weist das Kopfstück 3 an Rippen 36 ausgebildete Federanlageflächen 37 auf. Die Rippen 36 erstrecken sich in etwa sternförmig von der Buchse 31 zu der Innenfläche des Außenmantels 30. Dementsprechend wird zwischen der Innenfläche des Außenmantels 30, der Außenfläche der Führungsbuchse 31 und dem Federanlageflächen 37 ein zu der Unterseite des Kopfstückes 3 offener Ringraum 38 ausgebildet.

Das Kopfteil 3 ist zu der behälterwärtigen Seite des Außenmantels 30 offen und oberhalb dieser Stirnseite im wesentlichen nach Art einer Kappe ausgebildet. An der der Stirnseite des Außenmantels 30 abgewandten Oberseite des Kopfteiles 3 befindet sich eine Produktabgabeöffnung 39 des Ausgabekanals 32.

Das Kopfgegenstück 4 weist im wesentlichen zwei konzentrische Zylinderabschnitte aufweist, nämlich einen äußeren Haltezylinder 41 und einen im Durchmesser kleineren Führungszylinder 42. Der Haltezylinder 41 überragt den Führungszylinder 42 auf der dem Behälter 1 zugewandten Seite, wohingegen der Führungszylinder 42 den Haltezylinder 41 auf der anderen Seite überragt. An der dem Behälter 1 abgewandten Stirnseite des Haltezylinders 41 ist ein radial von dort nach innen sich erstreckender Ringsteg vorgesehen, der etwa mittig an die Außenfläche des Führungszylinders 42 stößt.

Der Haltezylinder 41 weist an seiner behälterseitigen Stirnseite eine nach außen vorspringende umlaufende Ringschulter auf. Die behälterseitige Stirnseite des Führungszylinders 42 bildet einen Förderkolbenschlag aus.

Im zusammengebauten Zustand befindet sich der Förderkolben 51 des Druckkolbens 5 gleitverschieblich in der Innenhülse 13 des Behälters 1 und deckt somit stirnseitig die Förderkammer 100 ab. Das Kopfgegenstück 4 ist konzentrisch zu der Innenhülse 13 angeordnet und mit seinem Haltezylinder 41 topfförmig über die Innenhülse 13 geschoben. Die Ringschulter des Kopfgegenstücks 4 liegt an der dem Behälter 1 abgewandten Stirnseite der Abdeckung 10 an.

Die Ringschulter des Kopfgegenstückes 4 befindet sich etwa im Bereich des stirnseitigen Endes der Innenhülse 13. Der sich hieran radial nach innen anschließende Führungszylinder 42 umgibt Ende der Führungsbuchse 31 des Kopfteils 3. Radial innerhalb dieser Führungsbuchse 31 befindet sich der Förderschaft 50 mit seinem Schaftbereich 56 kleineren Durchmessers. Der Förderkolben 51 des Druckkolbens 5 ist gleitverschieblich an der Innenwandung der Innenhülse 13 angeordnet. Die ringförmige Anlagefläche des Förderkolbens 51 liegt stirnseitig an dem Förderkolbenanschlag des Führungszylinders 42 an. Hierdurch wird die von der Feder 7 auf das Kopfstück 3 ausgeübte Vorspannkraft gehalten, welche über die Anlage von Mitnehmerschulter 34 und Mitnehmerkranz 57 den Druckkolben 5 in einer Richtung weg von dem Behälter 1 vorspannt.

Zwischen der Förderkammer 100 und dem Innenraum 12 des Behälters 1 befindet sich ein in bekannter Weise ausgebildetes Behälterventil 20, das mit seiner Dichtungsscheibe 21 gegen den Ringkranz 15 der Abdeckung 10 anliegt und den Innenraum 10a gegenüber der Förderkammer 100 abdichtet.

Im nicht benutzen Zustand befindet sich der Spender in Ausgangsstellung (0).

Bei der Benutzung des Spenders drückt ein Benutzer das Kopfstück 3 in Richtung auf den Behälter 1. Aufgrund der Inkompressibilität des in der Förderkammer 100 und dem Förderkanal 50a enthaltenen Substanz verharrt der Druckkolben 5 in seiner Lage. Das Kopfstück 3 bewegt sich relativ zu dem Druckkolben 5 in Richtung auf den Behälter 1 zu. Die formschlüssige Anlage zwischen dem Mitnehmerkranz 57 und der Mitnehmerschulter 34 wird gelöst, bis die Schaftkappe 54 gegen die Innenfläche des Buchsenkopfes 35 stößt bzw. - je nach Ausgestaltung - die stirnseitige Druckfläche 33 am Ende der Führungsbuchse 31 an der ringförmigen Anlagefläche 51a des Förderkolbens 51 zur Anlage (Mittelposition M) kommt. Nach dieser axialen Verschiebung um den Verschiebeweg a liegen die Förderkanalauslassöffnungen 53 in dem Ausgabekanal 32 frei.

Das Kopfstück 3 wird bei dieser wie auch bei jeder übrigen axialen Relativbewegung zwischen dem Kopfstück 3 und dem Kopfgegenstück 4 bzw. zwischen dem Kopfstück 3 und dem Behälter 1 durch die Anlage der Außenumfangsfläche der Führungsbuchse 31 an der Innenumfangsfläche des Führungszylinders 42 gleitend geführt. Die Relativewegung zwischen dem Kopfstück 3 und dem Druckkolben 5 wird über die Anlage der Umfangsfläche des zweiten Schaftabschnittes an dem Schaftbereich 56 geführt.

Bei fortschreitender Druckbewegung des Kopfstückes 3 in Richtung auf den Behälter 1 wird der Druckkolben 5 mitgenommen. Hierbei verringert sich das Volumen der Förderkammer 100, so dass das sich in Förderrichtung hinter dem Behälterventil 30 befindende pastöse Produkt über die Förderkanalauslassöffnung 53 in den Ausgabekanal 32 abgegeben wird. Das pastöse Produkt verlässt den Ausgabekanal über dessen Produktabgabeöffnung 39.

Am Ende dieser Relativbewegung des Kopfstückes 3 in Richtung auf den Behälter 1 stoßen die behälterseitigen Dichtlippen 52 des Druckkolbens 5 gegen die Stirnseite des Ringspalts 16. In dieser Ausgabe-Endposition V hat die Förderkammer 100 ihr kleinstes Volumen erreicht.

Wird nunmehr das Kopfstück 3 von dem Benutzer freigegeben, so drückt die Schraubenfeder 7 das Kopfstück 3 in entgegengesetzter Richtung zurück. Hierbei verharrt zunächst der Druckkolben 5 in seiner Ausgabe-Endposition V. Lediglich das Kopfstück 3 bewegt sich weg von dem Behälter 1, und zwar so lange, bis die Mitnehmerschulter 34 zur Anlage an den Mitnehmerkranz 57 kommt.

Bei dieser axialen Verschiebung um die Wegstrecke a wird das in dem Ausgabekanal 32 befindliche pastöse Produkt in den hierbei gebildeten Raum zwischen der Schaftkappe 54 und der Innenseite des Buchsenkopfes 35 zurückgezogen. Das pastöse Produkt liegt danach am Ende dieser Verschiebewegung nicht mehr unmittelbar an der Produktausgabeöffnung 39 des Ausgabekanals 32 an, wodurch verhindert wird, dass pastöses Produkt am Ende des Fördervorgangs aus dem Ausgabekanal 32 tropft bzw. durch Verschmutzung im Bereich der Produktausgabeöffnung 39 beeinträchtigt wird.

Nach der Verlagerung um die Wegstrecke a und der Anlage von Mitnehmerkranz 57 und Mitnehmerschulter 34 wird auch der Druckkolben 5 bei fortschreitender Bewegung des Kopfstücks 3 in Richtung auf die Ausgangsstellung zurückbewegt, die dann erreicht wird, wenn der Förderkolbenanschlag gegen die ringförmige Anlagefläche des Förderkolbens 51 anliegt. Bei der Relativbewegung des Druckkolbens 5 weg von dem Behälter 1 wird pastöses Produkt aus dem Innenraum 10a des Behälters 1 durch die Behälteröffnung 11 in die Förderkammer 100 gefördert. Der hierbei in dem Innenraum 10a entstehende relative Unterdruck führt in an sich bekannter Weise zu einer Nachlaufbewegung des in dem Innenraum 12 befindlichen Nachlaufkolbens 22.

In Fig. 2 ist ein zweites Ausführungsbeispiel des schriftgemäßen Spenders gezeigt.

Gleiche Teile sind bei diesem Ausführungsbeispiel gegenüber dem vorher Diskutierten mit gleichen Bezugszeichnen gekennzeichnet. Der Behälter 1 des in Fig. 7 gezeigten Ausführungsbeispiels ist im Wesentlichen identisch wie der vorher beschriebene Behäiter ausgebildet mit einer Behälteraußenwand, die einen Innenraum 10a umgibt, in dem ein Nachlaufkolben 22 längsverschieblich angeordnet ist und welcher von einer Bodenplatte 2 verschlossen ist. Im Gegensatz zu dem vorher beschriebenen Ausführungsbeispiel weist der Behälter 1 an seiner stirnseitigen Abdeckung einen umlaufenden Rastring 17 auf. Das Kopfgegenstück 4 ist über die Ringschulter 44 radial nach außen verlängert und hat eine sich im Wesentlichen parallel zu dem Haltezylinder 51 erstreckende zylinderförmige Außenwand 46, deren Durchmesser größer als der Durchmesser des Außenmantels 30 des Kopfstücks 3 ist. Zwischen der Außenwand 46 und dem Haltezylinder 41 ist an der behälterwärtigen Unterseite des Kopfgegenstücks 4 eine Rastausnehmung 47 ausgeformt, welche mit dem Rastring 17 zur Ausbildung einer Rastverbindung zwischen dem Kopfstück 4 und dem Behälter 1 zusammenwirkt.

Bei dem in Fig. 2 gezeigten Ausführungsbeispiel ist das Kopfgegenstück 4 zusammen mit dem Kopfstück 3 als vorgefertigte Spenderkomponente ausgebildet. Das dem Behälter 1 abgewandte freie Ende der Außenwand 46 des Kopfgegenstücks 4 ist radial zur Ausbildung einer Rastnase 46a nach innen abgekröpft und überragt in axialer Richtung einen Ringwulst 30a, der an der Außenseite des Außenmantels 30 an dem Kopfstück 3 vorgesehen ist. Hierdurch ist ein Anschlag gebildet, durch welchen das Kopfgegenstück 4 unverlierbar mit dem Kopfstück 3 verbunden ist. Dieser Anschlag hält die von der Feder 7 aufgebrachten Federkräfte. Die das Kopfstück 3 und das Kopfgegenstück 4 umfassende Spenderkomponente kann somit vor der Montage auf den Behälter 1 vormontiert werden. Hierzu wird die Feder 7 in den Hohlraum zwischen dem Kopfstück 3 und dem Kopfgegenstück 4 eingesetzt. Die beiden Bauteile 3,4 werden so weit in axialer Richtung ineinander geschoben, bis der Ringwulst 30a an dem nach innen umbogenen Ende der Außenwand 46 vorbei geglitten ist.

Bei dem in Fig. 2 gezeigten Ausführungsbeispiel weist der Druckkolben 5 einen Mitnehmerkranz 57 auf, der an der Schaftkappe 54 ausgeformt ist. Dementsprechend dichtet der Mitnehmerkranz 57 bei der in Fig. 2 gezeigten Ausgangsstellung den Ausgabekanal 32 ab. Der Förderschaft 50 hat einen Schaftbereich 56 mit verkleinertem Durchmesser, dessen Längserstreckung der axialen Wegstrecke a entspricht. Dementsprechend wird die axiale Verschieblichkeit des Druckkolbens 5 gegenüber dem Kopfteil 3 durch die Schaftkappe 54 einerseits und die Längserstreckung des Schaftbereiches 56 mit verringertem Durchmesser andererseits festgelegt.

Bei dem in Fig. 2 gezeigten Ausführungsbeispiel besteht gegenüber dem vorerwähnten ersten Ausführungsbeispiel der weitere Unterschied, dass in dem Ausgabekanal 32 ein Verschlussdorn 32a vorgesehen ist, welcher einstückig an dem Kopfstück 3 ausgeformt ist. Durch den Verschlussdorn 32a wird die Produktabgabeöffnung 39 ringförmig. Die Produktabgabeöffnung 39 ist bei dem gezeigten Ausführungsbeispiel durch ein ringförmiges Verschließteil 60 abgedeckt, welches als separates Bauteil aus einem thermoplastischen Elastomer mit dem Kopfstück 3 verbunden ist. Das Verschließteil 60 liegt in der in Fig. 7 Ausgangsstellung an der äußeren Umfangsfläche und an Teilen der Stirnseite, insbesondere aber der Umfangsfläche des Verschlussdorns 32a an und dichtet somit den Ausgabekanal 32 ab. Einstückig mit dem Verschließteil 60 ist ein Überzug 61, der stoffidentisch wie das Verschließteil 60 ausgebildet ist und welcher sich über einen überwiegenden Teil der stirnseitigen Abdeckung des Kopfstücks 3 erstreckt. Durch diesen Überzug 61 wird eine rutschfeste Funktionsfläche an dem Kopfstück 3 ausgebildet.

Bei der Betätigung des in Fig. 2 gezeigten Spenders laufen die vorstehend insbesondere unter Bezugnahme auf die Fig. 1 erläuterten Vorgänge ab. Es besteht jedoch gegenüber dem vorerwähnten Ausführungsbeispiel vorliegend der Unterschied, dass beim Zurückstellen von Druckkolben 5 und Kopfstück 3 der Ausgabekanal gegenüber der Umgebung abgedichtet wird. Bei einer Relativbewegung des Druckkolbens 5 relativ zu dem Kopfstück 3 in Richtung auf den Behälter 1 zu, wird das in dem Ausgabekanal 32 befindliche Produkt - wie vorsteht bereits erwähnt - entgegen der Förderrichtung zurück in das Innere des Kopfstücks 3 gezogen. Bei dem in Fig. 2 dargestellten Ausführungsbeispiel bewirkt der hierbei entstehende Druckgradient zwischen der Atmosphäre und dem Ausgabekanal 32 ein vollständig dichtendes Anliegen des Verschließteiles 60 an den Flächen des Verschlussdornes 32a. Dementsprechend bleibt in dem Ausgabekanal 32 anliegendes pastöses Produkt nahezu unbeeinflusst von eventuellen Oxidationsvorgängen. Zusätzlich dichtet die Schaftkappe 54 den Förderkanal 50a gegenüber dem Ausgabekanal 32 ab, so dass insbesondere eine Beeinträchtigung in dem Förderkanal 50a befindlichen pastösen Produktes durch eventuell in den Ausgabekanal 32 eindringende Luft in jedem Fall vermieden wird.

Die beiden vorstehend beschriebenen Ausführungsbeispiele weisen gemeinsam den Vorteil auf, dass die Förderkanalöffnungen 58 erst nach einer Relativbewegung zwischen dem Kopfteil 3 und dem Druckkolben 5 in dem Ausgabekanal 32 freiliegen. Zum Fördern des pastösen Produktes aus der Förderkammer in Richtung auf die Produktabgabeöffnung 32a ist es nicht erforderlich, dass der zunächst aufgebaute Innendruck in der Förderkammer 100 dazu genutzt wird, ein in Förderrichtung dahinterliegendes Rückschlagventil zu öffnen. Dementsprechend kann das pastöse Produkt mit geringerem Kraftaufwand ausgefördert werden. Weiterhin bieten beide vorerwähnten Ausführungsbeispiele den Vorteil, dass das pastöse Produkt entgegen der Förderrichtung nach der Betätigung des Kopfstückes in dem Ausgabekanal 32 zurückgezogen wird, wobei das in Fig. 2 gezeigte Ausführungsbeispiel den zulässigen Vorteil hat, dass durch die dichtende Anlage des Verschließteiles 60 an dem Verschlussdorn 32a das in dem Spender enthaltene pastöse Produkt sicher vor einer Beeinträchtigung beispielsweise durch Sauerstoff in der Luft geschützt wird.

### Bezugszeichenliste

1 Behälter; 2 Bodenplatte; 3 Kopfstück; 4 Kopfgegenstück; 5 Druckkolben; 6 Verschlusskappe; 7 Schraubenfeder; 10 Abdeckung; 10a Innenraum; 11 Behälteröffnung; 11 a Haltesteg; 12 Außenhülse; 13 Innenhülse; 15 Ringkranz; 16 Ringspalt;17 Rastring 20 Behälterventil 21 Ventilscheibe; 22 Nachlaufkolben; 30 Außenmantel; 30a Ringwulst; 31 Führungsbuchse; 32 Ausgabekanal; 32a Verschlussdorn; 33 Druckfläche; 34 Mitnehmerschulter; 36 Rippe; 37 Federanlagefläche; 38 Ringraum; 39 Produktabgabeöffnung; 41 Haltezylinder; 42 Führungszylinder; 44 Ringschulter; 46 Außenwand; 46a Rastnase; 47 Rastausnehmung; 50 Förderschaft; 50a Förderkanal; 51 Förderkolben; 52 Dichtlippen; 53 Förderkanaleinlassöffnung; 54 Schaftkappe; 55 Zylinderabschnitt; 56 Schaftbereich; 57 Mitnehmerkranz; 58 Förderkanalauslassöffnung; 60 Verschießteil; 61 Überzug; 100 Förderkammer.

Solche Spender sind aber keineswegs in jedem Fall für kosmetische und/oder dermatologische Produkte geeignet: zwar hat dieser Spendertyp den Vorteil, dass Produktreste nicht aus der Spenderöffnung auslaufen. Dadurch wird eine unästhetische Verschmutzung des Spenders durch auslaufende Produktreste vermieden. Entscheidendes Merkmal des Spenders zum Erzielen dieses Effekts ist eine selbstverschließende Öffnung, die bei Betätigung der Pumpe geöffnet wird und im Ruhezustand verschlossen ist. Diese spezielle Bauart führt jedoch auch zu entscheidenden Problemen, die die Anwendbarkeit für kosmetische Produkte stark einschränkt.

Viele Produkte neigen dazu, in der Öffnung von Spendern durch Austrocknen zu verkleben bzw. einen harten Pfropfen zu bilden, da sie im Allgemeinen Wasser enthalten, das mit der Zeit verdampft. Beim beschriebenen Spender führt dies insbesondere bei unregelmäßiger Entnahme des Füllgutes zu besonders großen Problemen, da die Austrittsöffnung in Ruhestellung fast dicht ist, was dazu führt, dass die Funktionsfähigkeit bei längerem Stehenlassen nicht mehr gewährleistet ist. Beim Versuch das Produkt zu applizieren öffnet sich der Verschluss gar nicht oder nur teilweise..Dies tritt insbesondere dann auf, wenn Produkte im Nassbereich angewendet werden, weil sie dem Risiko der Verkeimung durch Spritzwasser ausgesetzt sind. Ein auf dem Verschluss wuchernder Mikrobenrasen wirkt auf den Anwender nicht nur Ekel erregend, sondern behindert auch die Beweglichkeit des Verschlusses, weil die Spalträume nicht mehr frei sind. Enthält der Spender Formulierungen, die filmbildende Polymere enthalten wie beispielsweise Haargestaltungszubereitungen, so tritt ein ähnlicher Effekt auf: der Verschlussmechanismus verklebt und die Spalträume werden mit Polymerablagerungen aufgefüllt. Dies führt zu Funktionsstörungen. Zur Lösung dieses Problems aber bietet die oben genannte Schrift keinen Hinweis.

Es hat sich für den Fachmann nicht vorhersehbar herausgestellt, dass ein kosmetisches Produkt umfassend einen Spender für pastöse Produkte mit einem das pastöse Produkt enthaltenden, im Wesentlichen zylindrischen Behälter (1), der bodenseitig einen unter dem Druck der Außenatmosphäre an einer Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, dadurch gekennzeichnet dass die Fördereinrichtung ein zu dem Behälter (1) und dem Kopfstück (3) längsverschiebliches Förderelement (5) umfasst, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlassöffnung (53) und eine Förderkanalauslassöffnung (58) aufweist, die durch eine Verschiebebewegung des Förderelementes (5) relativ zu dem Kopfstück (3) in eine Stellung bringbar ist, in der sich die Förderkanalauslassöffnung (58) zu dem Ausgabekanal (32) öffnet,
sowie eine wasserhaltige kosmetisch und/oder dermatologische Zubereitung, die ein Hilfsmittel zur Gängighaltung des Spenders gewählt aus der Gruppe der Polyole mit 2 bis 6 Kohlenstoffatomen und 2 bis 6 Hydroxy- oder Alkoxygruppen oder der Tenside, die die Oberflächenspannung des Produktes auf <30mN/m herabsetzen, den Mängeln des Standes der Technik abhelfen.

Beide Lösungen -Zusatz von Tensiden oder Polyolen führen dazu, dass das Produkt auch bei längerer Nichtbenutzung die Spenderöffnung nicht in einer Weise verklebt, die die Funktionsfähigkeit des Spenders einschränkt. Im Fall von Polyolen führen diese dazu, dass die Produkte auch beim teilweisen verdunsten des Produktwassers ihre Geschmeidigkeit behalten bzw. das ein Teil des Produktwassers durch die Polyole fest im Produkt gebunden wird, was ein vollständiges Austrocknen effektiv verhindert. Der Zusatz von Tensiden führt dazu, dass die Produktreste weniger gut mit der Oberfläche des Packmittels verkleben da durch eine geringere Oberflächenspannung das Produkt besser auf der Oberflächespreiten kann.

Es wurde weiter gefunden, dass es bevorzugt ist, wenn Förderkanalauslassöffnung (58) an der Umfangsfläche des Förderschaftes (50) ausgespart ist und dass das Kopfstück (3) eine die Förderkanalauslassöffnung (58) in der Ausgangstellung (0) der Fördereinrichtung abdeckende Buchse aufweist. Weiter ist es bevorzugt, wenn die Buchse als eine die Fördereinrichtung längsverschieblich führende Führungsbuchse (31) ausgebildet ist, die wenigstens eine mit der Umfangsfläche des Förderschaftes (50) zu sammenwirkende Führungsfläche aufweist.

Weiter ist es bevorzugt, wenn an dem Kopfstück (3) und der Fördereinrichtung Mitnehmermittel (34,57) vor gesehen sind, durch welche die Fördereinrichtung nach Handbetätigung bei Rückstellung des Kopfstücks (3) in die Ausgangsstellung (0) mitgenommen wird. Weiter ist es bevorzugt, wenn an der Buchse (31) eine Mitnehmerschulter (57) ausgebildet ist, die mit einem an dem Förderschaft (50) an geformten Mitnehmerkranz (34) zusammenwirkt. Besonders bevorzugt ist es, wenn die Mitnehmerschulter (34) endseitig an der Buchse (31) am Übergang zu dem Ausgabekanal (32) und der Mitnehmerkranz (57) im stirnseitigen Endbereich des Förderschaftes (50) vorgesehen sind. Weiter ist es bevorzugt, wenn der Förderkolben (51) den Förderschaft (50) zur Ausbildung einer ringförmigen Anlagefläche radial überragt und dass die Führungsbuchse (31) eine stirnseitige Druckfläche (33) aufweist, die in der Ausgangsstellung (0) mit axialem Abstand zu der Anlagefläche angeordnet und durch axiales Verschieben des Kopfstückes (3) in Richtung auf den Behälter (1) an die Anlagefläche anlegbar ist. Weiter ist es bevorzugt, wenn die Innenwand der Förderkammer (100) durch eine Innenhülse (13) gebildet ist, welche an der kopfstückseitigen Stirnseite des Behälters (1) an der dem Kopfstück (3) zu gewandten Seite an dem Behälter (1) vorgesehen ist. Weiter ist es bevorzugt, wenn der Spender ein Kopfgegenstück (4), das einen topfförmig auf die Innenhülse (13) gestülpten Haltezylinder (41) sowie einen konzentrisch zu dem Haltezylinder (41) angeordneten, die Gleiterschiebung des Kopfstückes (3) führenden Führungszylinder (42) aufweist. Besonders bevorzugt ist es, wenn das förderkammerseitige Ende des Führungszylinders (42) einen Förderkolbenanschlag für den Förderkolben (51) aufweist. Ganz besonders bevorzugt ist es, wenn der Haltezylinder (41) mit einer bodenseitigen Ringschulter (44) versehen ist, die eine Anlagefläche für eine das Kopfstück in der Ausgangsstellung (0) unter Vorspannung haltende Schraubenfeder ausbildet und auf die Stirnseite des Behälters (1) aufgesetzt ist. Weiter ist es bevorzugt, wenn das Kopfgegenstück (4) wenigstens einen Anschlag (46a) zur Begrenzung der axialen Verschiebebewegung des Kopfstücks (3) aufweist und zusammen mit dem Kopfstück (3) als vorgefertigte Spenderkomponente ausgebildet und stirnseitig an dem Behälter (1) befestigt ist. Dabei ist es bevorzugt, wenn die Spenderkomponente mit dem Behälter (1) über an dem Kopfgegenstück (4) und der Stirnseite des Behälters (1) ausgebildete Rastmittel (47 ; 17) verrastet ist. Weiter ist es bevorzugt, wenn das Kopfstück (3) derart längsverschieblich ist, dass das Kopfstück (3) mittels Handbetätigung von der Ausgangsstellung (0) zunächst um eine erste axiale Wegstrecke (a) zur Anlage an den Förderkolben bei gleichzeitiger Feilegung der Förderkanalauslassöffnung (58) in dem Ausgabekanal (32) in eine Mittelposition (M) bringbar ist und das Kopfstück (3) danach bei fortschreitender axialer Verschiebung unter Mitnahme des Förderkolbens (51) von der Mittelposition (M) in eine Ausgabe-Endposition (V) bringbar ist, in welcher die Förderkammer (100) durch Verschiebung des Förderkolbens (51) ihr kleinstes Volumen erreicht hat. Weiter ist es bevorzugt, wenn der Spender durch ein an dem Kopfteil befestigtes Verschließteil (60), durch welches eine Produktabgabeöffnung (39) des Ausgabekanals (32) verschließbar ist. Dabei ist es bevorzugt, wenn die Produktabgabeöffnung (39) ringförmig um einen in dem Ausgabekanal angeordneten Verschlussdorn (32a) ausgebildet ist und dass das Verschließteil (60) eine ringförmig ausgebildete, an den Verschlussdorn dichtend anlegbare Dichtlippe aufweist. Dabei ist es besonders bevorzugt, wenn das Verschließteil (60) aus einer weichelastischen Kunststoffmasse, vorzugsweise aus einem thermoplastischen Elastomer gebildet ist. Dabei ist es ganz besonders bevorzugt, wenn das Verschließteil (60) einstückig mit einem zumindest stirnseitig an der Außenseite des Kopfteiles (3) ausgebildeten Überzug (61) ist. Die Erfindung umfasst auch die Verwendung von beschriebenen Polyolen oder Tensiden in einem wasserhaltige kosmetische und/oder dermatologische Zubereitungen enthaltenden oben beschriebenen Spender als Hilfsmittel zur Gängighaltung des Spenders. Bei all dem ist es besonders bevorzugt, wenn das Polyol Glycerin oder Propylenglycol ist. Diese Polyole sind besonders gut zur Gängighaltung des Spenders geeignet, weil bei ihr molares Wasserbindungsvermögen besonders günstig ist. Der Gehalt an Polyol beträgt besonders bevorzugt mindestens 0,1 Gew. %, ganz besonders bevorzugt 0,5-20 Gew. %, ganz außergewöhnlich bevorzugt 1-10 Gew. %% bezogen auf das Gewicht der Zubereitung. Weiter ist es bevorzugt, wenn ein Tensid mit einem HLB-Wert von mindestens 10 verwendet wird. Weiter ist es besonders bevorzugt, wenn als Tensid ein Alkylethersulfat gewählt wird. Dies hat den Vorteil, dass aufgrund der Sulfatgruppe die Ionisierung und somit der tensidische Charakter nicht vom pH-Wert anbhängig ist und somit eine pH-Verschiebung beispielsweise durch Austrocknung die Wirkung des Hilfsmittels zur Gängighaltung des Spenders nicht beeinträchtigt. Weiter ist es bevorzugt, wenn sowohl Polyol, als auch Tensid enthalten sind. Weiter ist es bevorzugt, wenn das Verhältnis aus Polyol zu Wasser 1:3 bis 1:25 beträgt.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Es kann von Vorteil sein, wenn die Zubereitungen weitere Inhaltstoffe wie Emulgatoren, Lipide, Farbstoffe, Pigmente, Gelbildner, Polymere, Puderrohstoffe, Antioxidantien, Komplexbildner, Selbstbräuner, Lichtschutzmittel, Haarkonditioniermittel, kosmetische Wirkstoffe, Konservierungsstoffe und/oder Hautbefeuchtungsmittel enthalten.

Besonders vorteilhaft ist es, wenn noch weitere Polyole und/oder Tenside enthalten sind. Solche Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:
RNH₂⁺CH₂CH₂COOH X⁻ (bei pH=2) X⁻ = beliebiges Anion, z.B. Cl⁻
RNH₂⁺CH₂CH₂COO⁻ (bei pH=7)
RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind
Acylaminosäuren (und deren Salze), wie
   1. Acylglutamate, beispielsweise Natriumacylglutamat, Di-TEA-palmitoylaspartat und Natrium Caprylic/ Capric Glutamat,
   2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
   3. Sarcosinate, beispielsweise Myristoyl Sarcosin, TEA-lauroyl Sarcosinat, Natriumlauroylsarcosinat und Natriumcocoylsarkosinat,
   4. Taurate, beispielsweise Natriumlauroyltaurat und Natriummethylcocoyltaurat,
   5. Acyllactylate, Lauroyllactylat, Caproyllactylat
   6. Alaninate
Carbonsäuren und Derivate, wie
   1. Carbonsäuren, beispielsweise Laurinsäure, Aluminiumstearat, Magnesiumalkanolat und Zinkundecylenat,
   2. Ester-Carbonsäuren, beispielsweise Calciumstearoyllactylat, Laureth-6-Citrat und Natrium PEG-4-Lauramidcarboxylat,
   3. Ether-Carbonsäuren, beispielsweise Natriumlaureth-13-Carboxylat und Natrium PEG-6-Cocamide Carboxylat,
   Phosphorsäureester und Salze, wie beispielsweise DEA-Oleth-10-Phosphat und Dilaureth-4 Phosphat,
Sulfonsäuren und Salze, wie
   1. Acyl-isethionate, z.B. Natrium-/ Ammoniumcocoyl-isethionat,
   2. Alkylarylsulfonate,
   3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
   4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat,
   sowie
Schwefelsäureester, wie
   1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
   2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.
Als erfindungsgemäß vorteilhafte Polyole können erfindungsgemäß alle organischchemischen Verbindungen mit zwei oder mehr Alkoholfunktionen eingesetzt werden. Erfindungsgemäß bevorzugt ist der Einsatz von Sorbitol, Propylen Glycol, Dipropylenglycol sowie Butylen Glycol, Panthenol, Kohlenhydraten (z.B. Mono-, Di-, Oligo- und Polysaccharide wie Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist), Hydroxysäuren, insbesondere α-Hydroxysäuren wie Milchsäure und/oder Lactate, insbesondere Natriumlactat und/oder Citronensäure, Ethylhexyloxyglycerin. Das erfindungsgemäß besonders bevorzugte Polyol ist Glycerin.

Es ist bei all diesem im Einzelfalle möglich, dass die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so dass er weiß, dass bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### 1) Duschen und Bäder

| | **1** | **2** | **3** | **4** | **5** |
|---|---|---|---|---|---|
| Natrium Laurethsulfat | 11,0% | 9,0% | 9,5% | 9,5% | 9,5% |
| Natrium Myrethsulfat | | | | 3,0% | |
| Cocoamidopropylbetain | 3,5% | 4,5% | 4,5% | 3,0% | 3,5% |
| Natriumcocoylglutamat | 1,5% | 2,0% | 2,5% | 1,5% | 2,5% |
| PEG-40 hydriertes Rizinusöl | 0,5% | 0,5% | 0,5% | 0,5% | 0,5% |
| PEG-7 Glyceryl Cocoat | | 2,3% | | | 1,5% |
| Glycerin | | | | 0,5% | |
| PEG-200 hydriertes Glycerylpalmitat | 0,5% | 0,3% | | 0,5% | 0,1% |
| PEG-90 Glyceryl Isostearat | | 0,3% | 0,8% | | 0,5% |
| Laureth-2 | | 0,1% | 0,1% | | 0,1% |
| Hydroxypropyl Guarhydroxypropyltrimonium Chlorid | | | 0,2% | | |
| Natriumchlorid | 1,0% | 1,0% | | 2,0% | 1,0% |
| Trinatrium EDTA | 0,2% | | | 0,2% | |
| Tetranatrium Imminodisuccinat | | 0,8% | | | |
| Polyquaternium-10 | 0,2% | | | | 0,2% |
| Benzophenone-4 | | | | 0,1% | |
| Glycol Distearat | | 0,6% | | | |
| Glycerin | | 0,3% | | | |
| Laureth-4 | | 0,3% | | | |
| Styrene/Acrylate Copolymer | 1,0% | | 1,0% | | 1,0% |
| Alcohol denat. | | 1,0% | | | |
| Pflanzenextrakte | | | 0,2% | | 0,2% |
| Natürliche Öle | 0,2% | | | | |
| Konservierungsstoffe | q.s. | q.s. | q.s. | q.s. | q.s. |
| Farbstoffe | q.s. | q.s. | q.s. | q.s. | q.s. |
| Citronensäure | q.s. | q.s. | q.s. | q.s. | q.s. |
| Parfum | q.s. | q.s. | q.s. | q.s. | q.s. |
| Wasser | ad 100 | ad 100 | ad 100 | ad 100 | ad 100 |

| | **6** | **7** | **8** | **9** | **10** | **11** |
|---|---|---|---|---|---|---|
| Natrium Laurethsulfat | 8,5% | 9,0% | 12,0% | 6,5% | 8,0% | 6% |
| Cocoamidopropylbetain | 4,0% | 3,0% | 4,0% | 3,25% | 3,5% | 3,5% |
| Natriumcocoylglutamat | 2,5% | 2,0% | 0,5% | 0,2% | 0,5% | 0,5% |
| Decyl Glucoside | 1,5% | | 0,5% | 2,0% | 4,0% | 3% |
| Dinatrium PEG-5 Laurylcitrat Sulffosucinat | | 2,5% | | | | |
| PEG-40 hydriertes Rizinusöl | 0,5% | 0,5% | 0,5% | 0,5% | 0,5% | 0,5% |
| PEG-7 Glyceryl Cocoat | | | 0,3% | 0,2% | 0,2% | 0,2% |
| Glycerin | 1,0% | | | | | |
| Glyceryl Laurat | | | 0,2% | | | |
| PEG-200 hydriertes Glycerylpalmitat | 0,3% | 0,2% | 0,3% | 0,5% | | 2,5% |
| PEG-90 Glyceryl Isostearat | | 0,2% | | | 0,3% | 0,2% |
| Laureth-2 | | 0,1% | | | 0,1% | 0,1% |
| PEG-120 Methyl Glucose Dioleat | | | | 0,5% | | |
| Hydroxypropyl Guarhydroxypropyltrimonium Chlorid | | | | | 0,8% | |
| Natriumchlorid | 1,0% | 1,0% | 1,1% | 1,0% | | 0,5% |
| Trinatrium EDTA | 0,2% | | | | | 1,0% |
| Tetranatrium Imminodisuccinat | | 1,0% | | | | |
| Polyquaternium-10 | | | | | | |
| Benzophenone-4 | 0,1% | 0,1% | | | | |
| Glycol Distearat | | | 0,8% | 0,6% | | 0,6% |
| Glycerin | | | 0,4% | 0,3% | | 0,3% |
| Laureth-4 | | | 0,4% | 0,3% | | 0,3% |
| Styrene/Acrylate Copolymer | | | | | 1,0% | |
| Pflanzenextrakte | | | | 0,05% | | |
| Natürliche Öle | | | | | | 0,2% |
| Konservierungsstoffe | q.s. | q.s. | q.s. | q.s. | q.s. | q.s. |
| Farbstoffe | q.s. | q.s. | q.s. | q.s. | q.s. | q.s. |
| Citronensäure | q.s. | q.s. | q.s. | q.s. | q.s. | q.s. |
| Parfum | q.s. | q.s. | q.s. | q.s. | q.s. | q.s. |
| Wasser | ad 100 | ad 100 | ad 100 | ad 100 | ad 100 | ad 100 |

| | **12** | **13** | **14** | **15** | **16** | **17** | **18** | **19** |
|---|---|---|---|---|---|---|---|---|
| Sodium Laurethsulfat | 13,2% | 11% | 9,5% | 5% | 9,5% | 10% | 9,5% | 7% |
| Cocoamidopropylbetain | 1,65% | 3,3% | 3,8% | 2% | 3,8% | 3,8% | 3,8% | 3,8% |
| Natriumcocoylglutamat | 1,25% | 0,75% | 2,5% | 1,5% | 2,5% | 1% | 2,5% | 2,5% |
| Eumulgin HSP | 0,30% | 0,50% | 1% | 1,2% | 1% | 2% | 2% | 3% |
| PEG-7 Glyceryl Cocoat | - | 0,3% | - | - | 1% | - | - | - |
| PEG-90 Glyceryl I-sostearat | - | - | - | - | 0,5% | 0,5% | - | - |
| Laureth-2 | - | - | - | - | 0,05% | 0,05% | - | - |
| PEG-200 hydriertes Glycerylpalmitat | 0,50% | 0,50% | 0,5% | 0,7% | - | 0,1% | 0,5% | 0,5% |
| Polyquaternium-10 | 0,2% | - | 0,2% | 0,1% | 0,2% | 0,2% | 0,2% | 0,2% |
| Natriumbenzoate | 0,45% | 0,45% | 0,45% | 0,45% | 0,45% | 0,45% | 0,45% | 0,45% |
| Styrene acrylates copolymer | - | - | 0,3% | 0,2% | 0,3% | 0,3% | 0,3% | - |
| Natriumsalicylate | 0,20% | 0,20% | 0,2% | 0,2% | 0,2% | 0,2% | 0,2% | 0,2% |
| Citric acid | 0,50% | 0,50% | 0,5% | 0,5% | 0,5% | 0,5% | 0,5% | 0,5% |
| Tocopheryl acetate | 0,2% | - | - | - | - | - | - | - |
| Ricinus Communis | - | - | - | - | 0,3% | - | 0,5% | - |
| Cyclomethicone | - | - | 0,2% | - | - | 0,2% | 0,2% | 0,2% |
| Sodium Chloride | - | - | - | 0,3% | 0,25% | - | 0,3% | - |
| Parfum | 0,5% | 0,5% | 0,5% | 0,5% | 0,5% | 3% | 1,5% | 4% |
| Wasser | ad 100 | ad 100 | ad 100 | ad 100 | ad 100 | ad 100 | ad 100 | ad 100 |

| | **20** | **21** | **22** | **23** | **24** |
|---|---|---|---|---|---|
| Sodium Myrethsulfat | 5% | 4% | 6% | 12% | 2,5% |
| Laurylglucosid | 2,5% | - | - | 1% | 2,5% |
| Decylglucosid | - | 3% | - | - | - |
| Sodium Cocoamphoacetate | 6,5% | 7% | 8% | 3% | 4,5% |
| PEG-200 hydriertes Glycerylpalmitat | 0,4% | 0,4% | 0,4% | 0,6% | 0,4% |
| Eumulgin HSP | 1% | 1% | 1% | 0,5% | 0,8% |
| Diammonium Citrate | 0,12% | 0,12% | 0,12% | 0,12% | 0,12% |
| Polyquaternium-10 | 0,2% | - | - | 0,15% | 0,2% |
| Natriumbenzoate | 0,3% | 0,3% | 0,3% | 0,45% | 0,3% |
| Natriumsalicylate | 0,2% | 0,2% | 0,2% | 0,45% | 0,2% |
| Citric acid | 1,2% | 1,2% | 1,2% | 1 % | 1,2% |
| Persea gratissima | 0,3% | - | - | - | - |
| Paraffinum liquidum | - | 0,2% | - | - | - |
| Squalane | - | - | - | 0,1% | - |
| Parfum | 0,4% | 0,4% | 0,4% | 0,4% | 0,6% |
| Wasser | ad 100 | ad 100 | ad 100 | ad 100 | ad 100 |

| | **14** | **15** | **16** | **17** | **18** |
|---|---|---|---|---|---|
| Natrium Laurethsulfat | 13,2% | 11% | 9,5% | 5% | --- |
| Cocoamidopropylbetain | 1,65% | 3,3% | 3,8% | --- | --- |
| Natriumcocoylglutamat | 1,25% | 0,75% | 2,5% | 2% | 7% |
| Hydroxypropyl Stärke Phosphat Ester (Structure XL) | 20% | 8% | 4% | 3% | 2% |
| PEG-40 hydriertes Rizinusöl | 0,50% | 0,50% | 0,50% | 0,1% | 0,50% |
| PEG-100 hydriertes Glycerylpalmitat | 0,50% | 0,50% | 0,50% | --- | 0,50% |
| Polyquaternium-10 | 0,2% | --- | 0,2% | --- | 0,2% |
| Natriumbenzoat | 0,45% | 0,45% | 0,45% | 0,45% | 0,45% |
| Natriumsalicylat | 0,20% | 0,20% | 0,2% | 0,20% | 0,2% |
| Citronensäure | 0,50% | 0,50% | 0,5% | 0,50% | 0,5% |
| Parfum | q.s. | q.s. | q.s. | q.s. | q.s. |
| Wasser | ad 100 | ad 100 | ad 100 | ad 100 | ad 100 |

| | **19** | **20** | **21** | **22** | **23** |
|---|---|---|---|---|---|
| Natrium Myrethsulfat | 5% | 4% | 6% | --- | --- |
| Natriumcocoylglutamat | 2,5% | 1% | 5% | 3% | 1,8% |
| Decylglucosid | --- | 3% | --- | 3% | 2% |
| Natrium Cocoamphoacetat | 6,5% | 7% | 8% | --- | --- |
| Hydroxypropyl Stärke Phosphat Ester (Structure XL) | 2% | 0,8% | 5% | 2% | 0,3% |
| PEG-200 hydriertes Glycerylpalmitat | 0,4% | 0,4% | 0,4% | --- | --- |
| PEG-40 hydriertes Rizinusöl | 1 % | 1 % | 1 % | 0,5% | 0,5% |
| Diammonium Citrat | 0,12% | 0,12% | 0,12% | 0,12% | 0,12% |
| Polyquaternium-10 | 0,2% | --- | --- | --- | 0,2% |
| Natriumbenzoat | 0,3% | 0,3% | 0,3% | 0,3% | 0,3% |
| Natriumsalicylat | 0,2% | 0,2% | 0,2% | 0,2% | 0,2% |
| Citronensäure | 1,2% | 1,2% | 1,2% | 0,8% | 1% |
| Parfum | q.s. | q.s. | q.s. | q.s. | q.s. |
| Wasser | ad 100 | ad 100 | ad 100 | ad 100 | ad 100 |

### 2) Partikel- oder Feststoffhaltige Reinigungsprodukte

| | **1** | **2** | **3** | **4** | **5** | **6** | **7** |
|---|---|---|---|---|---|---|---|
| Natrium Laurethsulfat | 11,0 % | 9,5% | 12,0% | | 9,0% | 12% | |
| Natrium Myrethsulfat | | | | | | | 3,0% |
| Cocoamidopropylbetain | 4,0% | 4,5% | | | | | 4,0% |
| Natrium Cocoamphoactat | | | | | 4,5% | 3,5% | |
| Decyl Glucosid | | | 1,1% | 10,0% | | | |
| Lauryl Glucosid | | | | | | | 1,0% |
| Natriumcocoylglutamat | 1,5% | 2,5% | 0,8% | | 0,5% | 1,0% | |
| Acrylates Copolymer | 3,0% | 2,5% | 2,2% | | | | 1,7% |
| Acryltes/C10-30 Alkyl Acrylat Crosspolymer | | | | 1,0% | | | |
| Magnesium Aluminium Silicat | | | | | 3,0% | 2,3% | |
| Hydroxypropyl Guarhydroxypropyltrimonium Chlorid | | | | | 0,1% | 0,1% | |
| Polyquaternium-10 | | | | | | | 0,1% |
| PEG-6 Caprylic/Capric Glycerides | | | 1,0% | | | | |
| PEG-40 hydriertes Rizinusöl | 0,5% | 0,5% | 0,5% | 0,5% | 0,5% | 0,5% | 0,5% |
| Glycol Distearate | 0,3% | | | | | | |
| Glycerin | 0,2% | | | | | | |
| Laureth-4 | 0,2% | | | | | | |
| PEG-7 Glyceryl Cocoat | | 1,5% | | | | | |
| Styrene/Acrylate Copolymer | | | 1,0% | | | | |
| PEG-3 Distearat | | | | | 2,0% | 2,0% | |
| Butylenglykol | | | | 10,0% | | | |
| Propylenglycol | | | | 15,0% | | | |
| Trinatrium EDTA | | 0,2% | | | | 0,2% | 0,2% |
| Benzophenone-4 | | 0,1% | | | | 0,1% | 0,1% |
| Polyethylene | 1,5% | | | | 5% | 2% | |
| Pigmente | q.s. | q.s. | q.s. | q.s. | q.s. | q.s. | q.s. |
| Konservierungsstoffe | q.s. | q.s. | q.s. | q.s. | q.s. | q.s. | q.s. |
| Farbstoff | q.s. | q.s. | q.s. | q.s. | q.s. | q.s. | q.s. |
| Citronensäure | q.s. | q.s. | q.s. | q.s. | q.s. | q.s. | q.s. |
| NaOH | q.s. | q.s. | q.s. | q.s. | q.s. | q.s. | q.s. |
| Parfum | q.s. | q.s. | q.s. | q.s. | q.s. | q.s. | q.s. |
| Wasser | ad 100 | ad 100 | ad 100 | ad 100 | ad 100 | ad 100 | ad 100 |

| | **7** | **8** | **9** | **10** | **11** |
|---|---|---|---|---|---|
| Natrium Laurethsulfat | 2% | 1,2% | 2% | 5% | - |
| Methyl Cocoyltaurat | 0,6% | 0,6% | 0,6% | - | 0,6% |
| Acylglutamat | - | - | - | - | 1,5% |
| Decyl glucosid | - | 0,5% | - | 1% | 1,5% |
| Carbomer | 1,2% | 1,2% | 1,2% | 1,2% | 1,2% |
| PEG-40 hydriertes Rizinusöl | 1% | 1% | 1% | 0,5% | 0,8% |
| Natriumhydroxid | 0,5% | 0,5% | 0,5% | 0,5% | 0,5% |
| PEG-7 Glyceryl cocoate | - | - | - | 0,7% | 0,3% |
| Glycerin | 2,0% | 2,0% | 2,0% | 2,0% | 2,0% |
| Xanthan Gum | 0,25% | 0,25% | 0,25% | 0,25% | 0,25% |
| Polyethylen | - | - | 0,3% | - | - |
| Squalane | - | 0,2% | - | - | - |
| Prunus dulcis | 0,3% | - | 0,2% | 0,3% | - |
| Phenoxyethanol | 0,5% | 0,5% | 0,5% | 0,5% | 0,5% |
| Natrium Chloride | - | - | 0,2% | 0,3% | - |
| Parabens | 0,2% | 0,2% | 0,2% | 0,2% | 0,2% |
| Parfum | 0,4% | 0,4% | 0,4% | 0,4% | 0,6% |
| Wasser | ad 100 | ad 100 | ad 100 | ad 100 | ad 100 |

### 3) Flüssig Seifen

| | **1** | **2** | **3** | **4** | **5** |
|---|---|---|---|---|---|
| Natrium Laurethsulfat | 5,0% | 4,5% | 5,0% | 5,0% | 10,5% |
| Natrium Cocoamphoacetat | 5% | 5,5% | | 5% | |
| Cocoamidopropylbetain | | | 4,5% | | 4,5% |
| Natrium Lauroyl Sarcosinat | | | | | 1,6% |
| PEG-40 hydriertes Rizinusöl | 0,5% | 0,5% | 0,5% | 0,5% | 0,5% |
| PEG-7 Glyceryl Cocoat | 1,0% | 1,5% | 0,75% | 1,0% | |
| PEG-4 Rapeseedamid | | | | | 4,0% |
| PEG-9 Cocoglycerid | | | | | 1,6% |
| Glycerin | 1,5% | 1,0% | 2% | 1,5% | |
| PEG-200 hydriertes Glycerylpalmitat | | 0,5% | | | 1,5% |
| PEG-90 Glyceryl Isostearat | | 0,5% | | | |
| Laureth-2 | | 0,1% | | | |
| PEG-120 Methyl Glucose Dioleat | 0,5% | | 0,3% | 0,5% | |
| Hydroxypropyl Guarhydroxypropyltrimonium Chlorid | | | | | 0,3% |
| Natriumchlorid | | | 0,2% | | |
| Trinatrium EDTA | | 0,2% | | | |
| Tetranatrium Imminodisuccinat | | | 1% | | |
| Polyquaternium-10 | 0,1% | | | 0,1% | |
| Benzophenone-4 | | 0,1% | | | |
| Glycol Distearat | | | 0,3% | | |
| Glycerin | | | 0,2% | | |
| Laureth-4 | | | 0,2% | | |
| Styrene/Acrylate Copolymer | | | | 2,5% | |
| Konservierungsstoffe | q.s. | q.s. | q.s. | q.s. | q.s. |
| Farbstoffe | q.s. | q.s. | q.s. | q.s. | q.s. |
| Citronensäure | q.s. | q.s. | q.s. | q.s. | q.s. |
| Parfum | q.s. | q.s. | q.s. | q.s. | q.s. |
| Wasser | ad 100 | ad 100 | ad 100 | ad 100 | q.s. |

### 4) Ölhaltige Produkte

| | **1** | **2** | **3** | **4** | **5** |
|---|---|---|---|---|---|
| Paraffinöl | 46% | 14% | 20% | 20% | 25% |
| Sojaöl | 24,3% | 36% | 20% | 20% | 25% |
| Sonnenblumenöl | | | | | |
| Weizenkeimöl | | | | | |
| Rizinusöl | | | | | |
| Natriumlaurylethersulfat | 7,4% | 12,3% | 11% | 11% | 11% |
| MIPA Laureth Sulfat | | | | | |
| Laureth-4 | | | | | |
| Kokamid DEA | | | | | |
| TIPA Laureth Sulfat | | | | | |
| Acrylates/C10-C30 Alkyl Acrylate Crosspolymer | - | - | 1% | 1% | 0,8% |
| PEG-40 Sorbitan Perisostearat | | | | | |
| Propylen Glycol | | | | | |
| Benzophenon-3 | | | | | |
| BHT | | | | | |
| Poloxamer 101 | | | | | |
| Natriumhydroxid | - | - | 0,2% | 0,2% | 0,2% |
| Konservierungsstoffe | q.s. | q.s. | q.s. | q.s. | q.s. |
| Farbstoffe | q.s. | q.s. | q.s. | q.s. | q.s. |
| Parfum | q.s. | q.s. | q.s. | q.s. | q.s. |
| Wasser | ad 100 | ad 100 | ad 100 | ad 100 | ad 100 |

### 5) Conditioner-Shampoo mit Perlglanz (und Trübungsmittel Formel 5)

| | **1** | **2** | **3** | **4** | **5** |
|---|---|---|---|---|---|
| Natrium Laurylethersulfat | 9 | 9 | 9 | 9 | 9 |
| Cocamidopropyl Betain | 4 | 4 | 4 | 4 | 4 |
| Dinatrium PEG-5 Laurylcitrat Sulfosuccinat | 3 | 3 | 3 | 3 | 3 |
| Verdicker | 0.1 | 0.1 | 0.1 | 0.2 | 0,3 |
| Polyquaternium-10 | 0.3 | 0.1 | 0.1 | 0.3 | - |
| Guar Hydroxypropyl-Trimonium Chlorid | - | - | 0.1 | 0.2 | 0.2 |
| Perlglanz | 1.5 | 3 | 4 | 2 | 2,5 |
| Trübungsmittel | - | - | - | - | 0.5 |
| Iminodibersteinsäure | 0.1 | 0.2 | 0.1 | 0.5 | 0.5 |
| PEG-40 hydriertes Rizinusöl | 0.2 | 0.2 | 0.2 | 0.2 | 0.2 |
| Natrium Salicylat | 0.4 | 0.4 | 0.4 | 0.4 | 0.4 |
| Natrium Benzoat | 0.4 | 0.4 | 0.4 | 0.4 | 0.4 |
| Natriumchlorid | 0,9 | 1,0 | 1,2 | - | - |
| Zitronensäure | q.s. | q.s. | q.s. | q.s. | q.s. |
| Parfüm | q.s. | q.s. | q.s. | q.s. | q.s. |
| Wasser | ad 100 | ad 100 | ad 100 | ad 100 | ad 100 |

### 6) Klares Conditioning-Shampoo

| | **1** | **2** | **3** | **4** | **5** |
|---|---|---|---|---|---|
| Natrium Laurylethersulfat | 10 | 9 | 3.5 | 3.5 | 0.5 |
| Natrium Myrethsulfat | - | - | 3.5 | 3.5 | 3.0 |
| Cocamidopropyl Betain | 4 | 4.5 | 3 | - | - |
| Natrium Cocoamphoacetat | - | - | - | 2.5 | - |
| Dinatrium PEG-5 Laurylcitrat Sulfosuccinat | - | - | - | - | 2.5 |
| Decylglucosid | - | - | - | - | 4.5 |
| Verdicker | 0.1 | 0.1 | 0.1 | 0.1 | 0.5 |
| Polyquaternium-10 | 0.1 | 0.1 | 0.05 | 0.25 | 0.2 |
| Guar Hydroxypropyl-Trimonium Chlorid | - | 0.1 | - | - | 0.2 |
| Hydrolysiertes Seidenprotein | - | - | - | - | 0.3 |
| Iminodibersteinsäure | 0.1 | 0.1 | 0.2 | - | - |
| PEG-40 hydriertes Rizinusöl | 0.2 | 0.2 | 0.2 | 0.1 | 0.2 |
| Natrium Salicylat | - | - | 0.4 | - | |
| Natrium Benzoat | 0.5 | 0.5 | 0.4 | 0.4 | 0.4 |
| Benzophenon-4 | - | - | 0.1 | - | - |
| Zitronensäure | q.s. | q.s. | q.s. | q.s. | q.s. |
| Parfüm | q.s. | q.s. | q.s. | q.s. | q.s. |
| Wasser | ad 100 | ad 100 | ad 100 | ad 100 | ad 100 |

### 7) Mildes Baby Shampoo

| | **1** | **2** | **3** | **4** | **5** |
|---|---|---|---|---|---|
| Natrium Myristylethersulfat | 4 | 4 | 5 | 5 | 4 |
| Decylglukoside | 4 | 4 | 4 | 4 | 4 |
| Dinatrium PEG-5 Laurylcitrat Sulfosuccinat | 4 | 4 | 3 | 5 | 5 |
| PEG-80 Sorbitan Laurat | 2 | 1 | 1 | - | 0.5 |
| Verdicker | 0.1 | 0.1 | 0.1 | 0.2 | 0.3 |
| Polyquaternium-10 | 0.3 | 0.1 | 0.1 | 0.3 | - |
| Guar Hydroxypropyl-Trimonium Chlorid | - | - | 0.1 | 0.2 | 0.2 |
| Perlglanz | - | - | 4 | 2 | 2.5 |
| Trübungsmittel | - | - | - | - | 0.5 |
| Iminodibersteinsäure | - | 0.2 | 0.1 | 0.5 | 0.5 |
| PEG-40 hydriertes Rizinusöl | 0.2 | 0.2 | 0.2 | 0.2 | 0.2 |
| Natrium Salicylat | 0.4 | 0.4 | 0.4 | 0.4 | 0.4 |
| Natrium Benzoat | 0.4 | 0.4 | 0.4 | 0.4 | 0.4 |
| Natriumchlorid | 0.9 | 1.0 | 1.2 | - | - |
| Zitronensäure | q.s. | q.s. | q.s. | q.s. | q.s. |
| Parfüm | q.s. | q.s. | q.s. | q.s. | q.s. |
| Wasser | ad 100 | ad 100 | ad 100 | ad 100 | ad 100 |

### 8) Leave on Konditioner

| | **1** | **2** | **3** | **4** |
|---|---|---|---|---|
| Laureth-4 | 0,6 | 0,4 | 0,5 | 0.5 |
| Cocosnußfettsäureethylhexylester | 0,9 | 0,8 | 0,8 | 1 |
| C12-13 Alkyllactat | 0,5 | 0,7 | 2,5 | 1 |
| Acrylate/C10-30 Alkyl Acrylat-Crosspolymer | 0,9 | 0,7 | 1,0 | 0.8 |
| Hydroxypropyl Guarhydroxypropyltrimoniumchlorid | 0,1 | 0,3 | 0,2 | 0.2 |
| PVP/VA Copolymer | 4 | 4 | 3 | 5 |
| Propylenglycol | 3 | 5 | 6 | 7 |
| Palmitamidopropyltrimoniumchlorid | 0,4 | 0,3 | 0,5 | 0.5 |
| Parfum, Lösungsvermittler, Konservierung, Komlexierungsmittel, Puffer | 2 | 2 | 2 | 2 |
| Wasser | Ad.100 | Ad.100 | Ad.100 | Ad.100 |

### 9) Styling Gel

| | **1** | **2** |
|---|---|---|
| PVP/VA Copolymer | 5,0 | 5,0 |
| Carbomer | 0,7 | 1,0 |
| Parfüm | 0,3 | 0,2 |
| PEG-40 hydriertes Rizinusöl | 0,5 | 0,3 |
| NaOH | q.s. | q.s. |
| Glycerin | 0,5 | 0,2 |
| Ethanol | 10,0 | - |
| Wasser | Ad 100 | Ad 100 |

### 10) Styling Emulsion

Viskositäten: über 300000 mPas

| | **1** | **2** | **3** | **4** |
|---|---|---|---|---|
| Paraffinöl | 5,0 | 5,0 | 5,0 | 5,0 |
| Sorbitan Stearat | 3,0 | 2,0 | 3,0 | 2,0 |
| Oleth-15 | 1,5 | 2,2 | 1,5 | 2,2 |
| PVP | 1,5 | 1,3 | | |
| Polyquaternium-46 | - | - | 4,0 | 3,5 |
| Glycerin | 5,0 | 6,0 | 5,0 | 6,0 |
| Konservierungsmittel | 0,4 | 0,4 | 0,4 | 0,4 |
| Parfüm | 0,2 | 0,2 | 0,2 | 0,2 |
| Wasser | Ad 100 | Ad 100 | Ad 100 | Ad 100 |

### 11) O/W Emulsionen

| | **1** | **2** | **3** | **4** | **5** | **6** | **7** |
|---|---|---|---|---|---|---|---|
| PEG-40 Castor Oil, Natrium Cetearyl Sulfat Cetearyl Alkohol | | | | | | | 2,50 |
| Glycerinmonostearat (SE) | 1,00 | 2,00 | 3,00 | 1,00 | | 1,50 | |
| Glyceryl Stearat Citrat | | | | | 2,00 | | |
| Stearinsäure | 3,00 | | 2,50 | 2,00 | | | |
| PEG-40 Stearat | | 2,00 | | | | 2,00 | |
| PEG-100 Stearat | | | 0,75 | | | | |
| Lauryl Methicon Copolyol | | | | 0,75 | | 0,50 | |
| Sorbitan Stearat | | | 0,75 | | | | |
| Cetyl Phosphat | | | 0,75 | | | | |
| Stearyl Alkohol | | | 3,00 | | 2,00 | 2,00 | 0,50 |
| Cetyl Alkohol | 1,00 | 2,00 | | | 0,50 | | 2,00 |
| UVASorb® K2A | | | | 4,00 | | 5,00 | |
| Uvinul® A Plus | 2,50 | | | 0,25 | 1,00 | 0,50 | |
| Butylmethoxydibenzoylmethan | | | | | | | 4,00 |
| 4-Methylbenzylidencampher | | | 3,00 | | | | |
| Bis-Ethylhexyloxyphenol Methoxyphenyl Triazin | 1,00 | | | | | 1,00 | 0,50 |
| Dinatrium Phenyl Dibenzimidazol Tetrasulfonat | | | | 1,00 | | 2,00 | |
| Phenylbenzmidazol Sulfonsäure | | | | | | | 3,00 |
| Ethylhexyl Triazon | | | | 2,00 | | 2,00 | |
| Diethylhexyl Butamido Triazon | 2,00 | | | | | | |
| Ethylhexyl Methoxycinnamat | 3,50 | | | 10,00 | | | |
| Octocrylen | | | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | 0,50 | 3,00 | | | | 5,00 |
| Drometrizol Trisiloxan | | | 0,50 | | | 1,00 | |
| Terephtalidene Dicampher Sulfonsäure | | | | | | 2,00 | |
| Dimethylcodiethylbenzalmalonat | | | | | | 3,00 | |
| Titandioxid T 805 | 2,00 | | | | 1,00 | 0,50 | |
| Titandioxid MT-100Z | | | | 3,00 | 1,00 | | |
| Zinkoxid Z-Cote HP1 | | | | | | | |
| C₁₂₋₁₅ Alkyl Benzoat | 2,50 | | | | | 7,00 | 5,00 |
| Dicaprylylether | | | 3,50 | | 2,00 | | |
| Caprylsäure/Caprinsäuretriglycerid | | | | | | | |
| Paraffinöl | | 6,00 | | | | | |
| Butylenglycol Dicaprylat/ Dicaprat | | | | 5,00 | 3,00 | | |
| Cetearyl Isononanoat | 4,00 | | | | | 2,00 | 2,00 |
| Dimethicon | 0,50 | 3,00 | 1,00 | | 2,00 | | |
| Cyclomethicon | | 3,00 | | 4,50 | | | 0,50 |
| Dimethicon / Vinyl Dimethicon Crosspolymer | 4,00 | | | | | | 0,50 |
| PVP Hexadecen Copolymer | | | | 0,50 | 1,00 | | 1,00 |
| Glycerin | 7,50 | 10,00 | | 7,50 | 5,00 | | 20,00 |
| Xanthangummi | | 0,20 | 0,05 | | | | 0,30 |
| Butylenglycol | 5,00 | | | | | 7,00 | |
| Ascorbinsäure | | | | | | 3,50 | |
| Tocopherol | 0,20 | | | | | | |
| Taurin | 1,00 | | | | | | |
| Vitamin E Acetat | | 0,40 | 0,25 | 0,50 | 0,75 | | 1,00 |
| Retinol | | | | | 0,05 | | |
| Dioic Acid (Octadecendicarbonsäure) | | | | 0,20 | | 0,25 | |
| Pyridoxin | | 0,5 | | | | | |
| Fucogel® 1000 | | | 1,50 | | | 5,00 | |
| Dihydroxyaceton | | 5,00 | | | | | |
| DMDM Hydantoin | 0,60 | | 0,40 | 0,20 | | | |
| Methylparaben | | 0,10 | 0,25 | | 0,50 | | |
| Phenoxyethanol | 0,40 | 0,50 | | 0,40 | 0,50 | | 0,60 |
| EDTA | 0,20 | | 0,35 | 0,50 | 0,02 | | 0,03 |
| Ethanol | 2,00 | | 1,50 | | 3,00 | 5,00 | 1,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| pH-Wert | 6,0-7,5 | 4,5-7,0 | 6,5-8,5 | 5,0-7,0 | 6,0-8,0 | 4,0-6,0 | 5,0-7,5 |

### O/W Emulsionen

| | **8** | **9** | **10** | **11** | **12** |
|---|---|---|---|---|---|
| Glycerinmonostearat (selbstemulgierend) | | | | 1,00 | |
| Glyceryl Stearat Citrat | 2,00 | 2,00 | 1,50 | | |
| Polyglyceryl-3- Methylglucose Distearat | | | | | 4,50 |
| Stearyl Alkohol | 2,00 | 2,00 | | | |
| Cetyl Alkohol | | | 2,00 | | 4,50 |
| UVASorb® K2A | | | | | |
| Uvinul® A Plus | | | | | |
| Bis-Ethylhexyloxyphenol Methoxyphenyl Triazin | 2,00 | 2,00 | 1,50 | | |
| Dinatrium Phenyl Dibenzimidazol Tetrasulfonat | | | | | |
| Ethylhexyl Triazon | | | | | |
| Diethylhexyl Butamido Triazon | | 1,00 | 2,00 | | |
| Ethylhexyl Methoxycinnamat | 2,00 | 6,00 | 5,00 | | |
| Octocrylen | 2,00 | 9,00 | | | |
| Methylen Bis-Benztriazolyl Tetramethylbutylphenol | | | | | |
| Ethylhexylsalicylat | | | | | |
| Drometrizol Trisiloxan | | | | | |
| Titandioxid T 805 | | 3,00 | 2,00 | | |
| C₁₂₋₁₅ Alkyl Benzoat | | | 3,00 | 1,00 | 1,00 |
| Hydriertes Kokosfettsäureglycerid | 1,00 | 1,00 | 3,00 | | |
| Dicaprylylether | 5,00 | 2,00 | 6,00 | | |
| Octyldodecanol | 6,00 | 5,00 | 4,00 | 3,00 | 4,00 |
| Butylenglycol Dicaprylat/ Dicaprat | 5,00 | 2,00 | | | |
| Caprylsäure/Caprinsäuretriglycerid | | | | 2,00 | 5,50 |
| Dimethicon | | | 2,00 | | |
| Cyclomethicon | 2,00 | 1,00 | | 3,00 | |
| Sorbitol | | | | 2,50 | |
| Acrylat/C10-30 Alkyl Acrylat Crosspolymer | 0,10 | 0,10 | 0,05 | | |
| PVP Hexadecen Copolymer | 0,50 | 0,50 | | | |
| **Glycerin** | **8,00** | **6,00** | **5,00** | **3,00** | **3,00** |
| Xanthangummi | 0,40 | 0,40 | 0,25 | 0,30 | 0,10 |
| **Butylenglycol** | | | **3,00** | **3,00** | |
| Linolsäure | 1,00 | | | | |
| Nachtkerzenöl | | 1,00 | | | |
| Genistein | 0,05 | | | | |
| Silymarin | | 0,1 | | | |
| Phosphatidylcholin | | | | 0,06 | |
| Silybin | | | | 0,03 | |
| Carnitin | | | 0,5 | | |
| Vitamin E Acetat | 0,50 | | 0,30 | 0,40 | 0,40 |
| Dihydroxyaceton | | | | 5,00 | 4,00 |
| DMDM Hydantoin | 0,60 | 0,60 | 0,50 | | |
| Methylparaben | | | | 0,30 | 0,30 |
| Phenoxyethanol | 0,40 | 0,40 | 0,35 | 0,50 | 0,50 |
| EDTA | 1,00 | 1,00 | 1,00 | | 1,00 |
| Ethanol | | | 3,00 | 3,00 | |
| Insekt Repellent 3535 | | | | | |
| Parfüm | 0,40 | 0,40 | 0,40 | 0,40 | 0,40 |
| Wasser | ad 100 | ad 100 | ad 100 | ad 100 | ad 100 |
| Neutralisationsmittel (Natriumhydroxid, Kaliumhydroxid) | qs | qs | qs | qs | qs |
| pH-Wert | 5,0-7,0 | 5,0-7,0 | 5,0-7,0 | 5,0-7,0 | 5,0-7,0 |

### O/W-Emulsionen

| | **13** | **14** | **15** | **16** | **17** | **18** | **19** |
|---|---|---|---|---|---|---|---|
| Glyceryl Stearat, Ceteareth-12, Ceteareth-20, Cetearyl Alcohol, Cetyl Palmitat | | | | | | | 1,50 |
| Glycerinmonostearat (SE) | | 4,00 | | | | | |
| Glyceryl Stearat Citrat | 2,00 | | | | | | |
| Polyglyceryl-3-Methylglucose Distearat | | | | | 2,00 | | |
| Cetearyl Glucosid & Cetearyl Alkohol | | | | | | 2,00 | |
| Triceteareth-4 Phosphat | | | 1,20 | | | | |
| Trilaureth-4 Phosphat | | | | 2,00 | | | |
| Ceteareth-6 | | | 0,50 | 0,50 | | | |
| Sorbitan Stearat | | | 0,75 | 1,00 | 1,00 | | |
| Cetyl Phosphat | | | | | 2,00 | | |
| Stearyl Alkohol | | 2,50 | 3,00 | | | | 1,50 |
| Cetyl Alkohol | 2,50 | 1,00 | | | 0,50 | 2,00 | 2,00 |
| UVASorb® K2A | 1,00 | | | 4,00 | | 5,00 | |
| Uvinul® A Plus | 3,00 | 2,50 | 0,50 | 0,25 | 1,00 | 0,50 | |
| Butylmethoxydibenoylmethan | | | | | | | 4,50 |
| Bis-Ethylhexyloxyphenol Methoxyphenyl Triazin | | 2,00 | | | | 1,00 | 0,50 |
| Dinatrium Phenyl Dibenzimidazol Tetrasulfonat | | | | 1,00 | | 2,00 | |
| Phenylbenzmidazol Sulfonsäure | | | | | 3,00 | | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | | | | | | 5,00 |
| Drometrizol Trisiloxan | | | 0,50 | | | 1,00 | |
| Terephtalidene Dicampher Sulfonsäure | | 3,00 | | | | | |
| Dimethylcodiethylbenzalmalonat | | | 5,00 | | | | |
| Titandioxid MT-100Z | 1,00 | | | 3,00 | 1,00 | | |
| Zinkoxid Z-Cote HP1 | | | | | | 3,00 | |
| Corapan TQ® | | | | | | | 6,00 |
| C₁₂₋₁₅ Alkyl Benzoat | | 2,50 | | | | 7,00 | 5,00 |
| Dicaprylylether | | | 3,50 | | 2,00 | | |
| Butylenglykol Dicaprylat/ Dicaprat | 5,00 | | | 5,00 | 3,00 | | |
| Cetearyl Isononanoat | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| PVP Hexadecen Copolymer | 0,50 | | | 0,50 | 1,00 | | 1,00 |
| Glycerin | 3,00 | 7,50 | | 7,50 | 5,00 | | 20,00 |
| Xanthangummi | 0,15 | | 0,05 | | | | 0,30 |
| Butylenglycol | 7,00 | 5,00 | | | | 7,00 | |
| Süßholzextrakt | | | | | | 0,5 | |
| Licochalcon | | | 0,05 | | | | |
| Curcumin | 0,05 | | | | | | |
| beta-Carotin | | | | 0,1 | | | |
| Ceramid III | | | | | 0,3 | | |
| Isoserinol | | 1,0 | | | | | |
| Vitamin E Acetat | 0,50 | | 0,25 | 0,50 | 0,75 | | 1,00 |
| Dioic Acid | 0,25 | | | 0,20 | | 0,25 | |
| Fucogel® 1000 | | | 1,50 | | | 5,00 | |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Alkohol | | 2,00 | 1,50 | | 3,00 | 5,00 | 1,00 |
| Insekt Repellent 3535 | | | 5,00 | | | | |
| Meersalz | 0,1 | | | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (NaOH, KOH) | qs | qs | qs | qs | qs | qs | qs |
| pH-Wert | 4,5-6,0 | 4,5-7,0 | 5,5-7,5 | 5,0-7,0 | 5,5-7,5 | 4,0-7,0 | 4,0-7,5 |

### 12) Schaumförmige O/W- Emulsionen:

| | **1** | | **2** | |
|---|---|---|---|---|
| | Gew.-% | Vol.-% | Gew.-% | Vol.-% |
| Stearinsäure | 5,00 | | 1,00 | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| Caprylsäure/Caprinsäuretriglycerid | 4,00 | | 2,00 | |
| C12-15 Alkyl Benzoat | 10,00 | | 15,50 | |
| Cyclomethicon | 4,00 | | | |
| Dimethicon | | | 0,50 | |
| Octylisostearat | | | 5,00 | |
| Myristyl Myristat | | | 2,00 | |
| Ceresin | 1,50 | | | |
| Glycerin | 5,00 | | 10,00 | |
| UVASorb® K2A | 2,00 | | | |
| Uvinul A Plus® | 2,00 | | 1,50 | |
| Terephthaliden Dicamphersulfonsäure | 0,50 | | | |
| Drometrizol Trisiloxan | 1,50 | | | |
| Ethylhexylmethoxycinnamat | 5,00 | | 4,00 | |
| Ethylhexyltriazon | | | 3,00 | |
| Octocrylene | 5,00 | | | |
| Titandioxid Uvinul T 805 | 1,00 | | | |
| BHT | | | 0,02 | |
| Na₂H₂EDTA | 0,50 | | 0,10 | |
| Parfüm, Konservierungsmittel, | q.s. | | q.s. | |
| Farbstoffe, usw. | q.s. | | q.s. | |
| Kaliumhydroxid | q.s. | | q.s. | |
| Wasser | ad 100,00 | | ad 100,00 | |
| | pH-Wert eingestellt auf 6,5-7,5 | | pH-Wert eingestellt auf 5,0-6,0 | |
| Emulsion 1 | | 70 | | |
| Emulsion 2 | | | | 35 |
| Gas (Stickstoff, Sauerstoff oder Kohlendioxid) | | 30 | | |
| Gas (Luft) | | | | 65 |

| **Emulsion** | **3** | **4** | **5** |
|---|---|---|---|
| Stearinsäure | 2,00 | 2,00 | |
| Palmitinsäure | | | 1,50 |
| Cetylalkohol | 2,50 | 2,00 | |
| Stearylalkohol | | | 3,00 |
| PEG-100 Stearat | | | 3,50 |
| PEG-40 Stearat | | 2,00 | |
| PEG-20-Stearat | 3,00 | | |
| Sorbitanstearat | | 0,80 | |
| C12-15 Alkyl Benzoat | 5,00 | | |
| C12-13 Alkyl Tartrat | | | 7,00 |
| Butylenglycol Dicaprylat/Dicaprat | | 6,00 | |
| Dicaprylyl Ether | | | 2,00 |
| Cyclomethicon | | 2,00 | 3,00 |
| Butylenglycol | 1,00 | | |
| Isohexadecan | 2,00 | | |
| Methylpropandiol | | | |
| Propylenglykol | | | 5,00 |
| Glycerin | 5,00 | 7,00 | |
| UVASorb® K2A | | | 2,00 |
| Uvinul A Plus® | 2,00 | | |
| NeoHeliopan® AP | | | |
| Phenylbenzimidazol Sulfonsäure | | | |
| Ethylhexylmethoxycinnamat | | | |
| Ethylhexyltriazon | 2,00 | 2,00 | 2,00 |
| Octocrylen | 2,00 | | |
| Bis-Ethylhexyloxyphenol Methoxyphenyltriazin | | 3,00 | 3,00 |
| Vitamin E Acetat | | 0,5 | |
| BHT | | | 0,10 |
| Na₂H₂EDTA | 0,50 | | |
| Parfüm, Konservierungsmittel | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

| **Emulsion** | **6** | **7** | **8** | **9** |
|---|---|---|---|---|
| Stearinsäure | 1,50 | | | |
| Palmitinsäure | | | 3,00 | 3,00 |
| Cetylalkohol | | 3,00 | | |
| Cetylstearylalkohol | | | 2,00 | 2,00 |
| Stearylalkohol | 3,00 | | | |
| PEG-100 Stearat | | 4,00 | | |
| PEG-40 Stearat | 3,00 | | | |
| PEG-20-Stearat | | | 3,00 | 3,00 |
| Sorbitanstearat | 1,00 | | | |
| Tridecyl Trimellitate | | 5,00 | | |
| C12-15 Alkyl Benzoat | | | 3,00 | 3,00 |
| Butylenglycol Dicaprylat/Dicaprat | 8,00 | | | |
| Octyldodecanol | | 2,00 | | |
| Kokosfettsäureglycerid | | | | 2,00 |
| Dicaprylyl Ether | | | 2,00 | 2,00 |
| Cyclomethicon | | | | |
| Dimethicon | 1,00 | | 2,00 | 2,00 |
| Isohexadecan | | 3,00 | | |
| Methylpropandiol | | 4,00 | | |
| Propylenglykol | | | | |
| Glycerin | 5,00 | | 6,00 | 6,00 |
| NeoHeliopan® AP | | 2,00 | | |
| Phenylbenzimidazol Sulfonsäure | 1,00 | 4,00 | 1,00 | 1,00 |
| Ethylhexylmethoxycinnamat | 5,00 | | 4,00 | 4,00 |
| Ethylhexyltriazon | | | | |
| Diethylhexylbutamidotriazon | 1,00 | | | |
| Butyl Methoxydibenzoylmethan | 2,50 | | 2,00 | 2,00 |
| Bis-Ethylhexyloxyphenol Methoxyphenyltriazin | 2,00 | | | |
| Vitamin E Acetat | 0,20 | | 0,30 | 0,30 |
| BHT | | 0,05 | | |
| Na₂H₂EDTA | | | 0,40 | 0,40 |
| Parfüm, Konservierungsmittel | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Kaliumhydroxid | | | | q.s. |
| Wasser | ad 100,0 | ad 100,0 | ad 100,0 | ad 100,0 |

Zur Herstellung des Schaums werden 80-97 Vol.-% der Emulsion I mit 3-20 Vol.-% eines geeigneten Gases (z.B. Propan/ Butan, Druckluft, Stickstoff) aufgeschäumt.

### 13) W/O-Emulsionen

| | **1** | **2** | **3** | **4** | **5** |
|---|---|---|---|---|---|
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 4,00 | | | 2,00 |
| Cyclomethicon | 12,00 | 20,00 | | 30,00 | 15,00 |
| UVASorb® K2A | | | | 0,50 | |
| Uvinul® A Plus | | 2,00 | 0,50 | 4,00 | 0,25 |
| Bis-Ethylhexyloxyphenol Methoxyphenyl Triazin | 1,00 | | | 0,50 | |
| Dinatrium Phenyl Dibenzimidazol Tetrasulfonat | | 1,50 | | | 2,00 |
| Drometrizol Trisiloxan | | | | 1,00 | |
| 4-Methylbenzylidencampher | 4,00 | | | | |
| Methylen Bis-Benztriazolyl Tetramethylbutylphenol | | | | | 3,00 |
| Ethylhexyl Methoxycinnamat | 3,00 | 4,00 | | | 10,00 |
| Ethylheyl Salicylat | | | 5,00 | | 3,50 |
| Octocrylen | | 5,00 | | 4,00 | |
| Diethylhexyl Butamido Triazon | | 1,00 | | | 6,50 |
| Ethylhexyl Triazon | 3,00 | | | | 4,00 |
| Titandioxid MT-100 Z | | 0,50 | 1,00 | 1,50 | 0,50 |
| Zinkoxid Z-Cote | 2,00 | | | | 4,00 |
| Dicaprylyl Carbonat | 5,00 | | 15,00 | | 4,00 |
| Dihexyl Carbonat | | 10,00 | | | |
| C12-15 Alkyl Benzoat | 7,00 | | 10,00 | | |
| Mineral Öl | 10,00 | | | | 6,00 |
| Kokosfettsäureglycerid | | 2,00 | | 5,00 | |
| PVP Hexadecen Copolymer | | 0,75 | | | 0,40 |
| Glycerin | 5,00 | 12,50 | | 5,00 | 15,50 |
| Sorbitol | 5,00 | | 10,00 | | |
| α-Glucosylrutin | | | | | 0,15 |
| EDTA | | 0,15 | 0,03 | | 0,15 |
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Alkohol | 2,00 | | | 5,00 | 1,00 |
| Farbstoff, öllöslich | 0,02 | | | | |
| Parfüm | 0,30 | 0,45 | 0,35 | | 0,15 |
| Wassser | ad. 100 | ad. 100 | ad. 100 | ad. 100 | ad. 100 |

### 14) W/O Emulsionen (Cremes & Lotions)

| | **1** | **2** | **3** | **4** | **5** | **6** |
|---|---|---|---|---|---|---|
| Cetyldimethicon Copolyol | | | | | 4,00 | |
| Polyglyceryl-2-dipolyhydroxystearat | 5,00 | 4,50 | | | | 4,50 |
| Polyglyceryl-3-Diisostearat | 1,75 | 1,50 | | | | |
| PEG-30-dipolyhydroxystearat | | | 3,00 | 5,00 | | |
| Bis-Ethylhexyloxyphenol Methoxyphenyl Triazin | | | 3,00 | 1,50 | | |
| Butyl Methoxydibenzoylmethan | | | 4,00 | | | |
| UVASorb® K2A | | | | 2,00 | 2,50 | |
| Uvinul ® A Plus | 3,00 | 1,00 | | 3,00 | 0,25 | 2,50 |
| Phenylbenzmidazol Sulfonsäure | | 4,00 | | | 2,00 | 0,50 |
| Ethylhexyl Methoxycinnamat | | 8,00 | | | 5,00 | 4,00 |
| Diethylhexyl Butamido Triazon | 3,00 | 1,00 | | 1,00 | | 3,00 |
| Ethylhexyl Triazon | | | 4,00 | 2,00 | 4,00 | |
| Octocrylen | 7,00 | | | | | 2,50 |
| Drometrizol Trisiloxan | | | | | | |
| Titandioxid Uvinul® T 805 | 2,00 | 1,00 | 5,00 | | | |
| Titandioxid MT-100 TV | | | | | | 2,00 |
| Zinkoxid Z-Cote® HP1 | 2,50 | | | 3,00 | | |
| Corapan TQ® | | | 6,00 | | | |
| Mineralöl | | | 6,00 | 5,00 | | 8,00 |
| Kokosfettsäureglycerid | 4,00 | 6,50 | | 5,00 | | |
| C12-15 Alkyl Benzoat | | | 9,00 | 8,00 | 9,00 | |
| Dicaprylylether | 10,00 | | | | | 7,00 |
| Butylenglycol Dicaprylat/Dicaprat | | | 9,00 | 7,00 | 8,00 | 4,00 |
| Cyclomethicon | 2,00 | | | | | 2,00 |
| PVP Eicosene Copolymer | 0,50 | | | | 1,50 | 1,00 |
| Trinatrium EDTA | 1,00 | | 1,00 | 0,50 | 0,35 | |
| Ethylhexyloxyglycerin | | 0,30 | | | | 0,50 |
| Methylpropandiol | | | | | | 7,50 |
| Glycerin | 5,00 | 7,50 | 6,00 | 8,00 | 7,50 | 2,50 |
| Butylenglykol | | 2,50 | | | | |
| Glycin Soja | | 1,00 | | | | |
| MgSO₄ | 1,00 | 0,50 | 0,30 | 0,30 | 0,50 | |
| Milchsäure & Natriumsalz der Milchsäure | 1,00 | 0,50 | | | | 0,85 |
| Vitamin E | 0,50 | | 0,50 | 1,00 | | 1,00 |
| DMDM Hydantoin | | 0,60 | | | 0,20 | |
| Methylparaben | 0,50 | | | | 0,15 | |
| Phenoxyethanol | 0,50 | 0,40 | 0,50 | 0,60 | 1,00 | 0,60 |
| Dihydroxyaceton | | | | | 5,50 | |
| Alcohol | 3,00 | | 2,00 | 3,00 | | 1,00 |
| Parfüm | 0,20 | | 0,20 | 0,20 | | 0,20 |
| Wasser | ad 100 | ad 100 | ad 100 | ad 100 | ad 100 | ad 100 |

### 15) Hydrodispersionen

| | **1** | **2** | **3** | **4** | **5** | **6** |
|---|---|---|---|---|---|---|
| | | | | | | |
| Glyceryl Stearat Citrat | | 0,40 | | | | |
| Cetyl Alkohol | | | | | 2,00 | |
| Natrium Carbomer | | | | | 0,30 | |
| Acrylates/C10-30 Alkyl Acrylate Crosspolymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Crosspolymer | | | | 5,00 | | 3,00 |
| UVASorb® K2A | | | | | 3,50 | |
| Uvinul® A Plus | 0,25 | | | 0,50 | 2,00 | 1,50 |
| Butyl Methoxydibenzoylmethan | | | 3,50 | | | |
| Bis-Ethylhexyloxyphenol Methoxypheny Triazin | | 2,00 | | 0,25 | | |
| Terephthaliden Dicampher Sulfonsäure | | | | | | 0,50 |
| Dinatrium Phenyl Dibenzimidazol Tetrasulfonat | 0,75 | | | | | 1,00 |
| Phenylbenzimidazol Sulfonsäure | | | 2,00 | | | |
| Ethylhexyl Methoxycinnamat | | | 7,00 | | 5,00 | 8,00 |
| Methylen Bis-Benzotriazolyl Tetramethylbutylphenol | | | | | | |
| Butyl Methoxydibenzoylmethan | | 3,50 | | | | |
| Diethylhexyl Butamido Triazon | | | 2,00 | 2,00 | | |
| Ethylhexyl Triazon | 4,00 | 3,00 | | | 4,00 | |
| Octocrylen | | | | 10,00 | | 2,50 |
| Titandioxid MT-100 Z | 0,50 | 2,00 | 1,00 | 2,00 | 3,00 | 1,00 |
| C₁₂₋₁₅ Alkyl Benzoat | 2,00 | | 2,50 | | | |
| C18-36 Triglycerid Fettsäure | | | 1,00 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| Lanolin | | | | | 0,35 | |
| PVP Hexadecen Copolymer | 0,50 | | 0,50 | | 0,50 | 1,00 |
| Ethylhexyloxyglycerin | | 0,75 | | 1,00 | | 0,50 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| α-Glycosylrutin | | | | | 0,25 | |
| Trinatrium EDTA | | 1,00 | 1,00 | 0,10 | 0,20 | |
| lodopropinylbutylcarbamat | 0,20 | 0,10 | | | | 0,15 |
| Methylparaben | 0,50 | | 0,20 | | 0,15 | |
| Phenoxyethanol | 0,50 | 0,40 | 0,40 | | 1,00 | 0,60 |
| Ethanol | 3,00 | 10,00 | 4,00 | 3,50 | | 1,00 |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |
| pH-Wert | 4,5-5,5 | 5,0-7,0 | 5,0-7,0 | 5,0-7,0 | 4,0-6,0 | 5,0-7,5 |

### 16) Gelcremes

| | **1** | **2** | **3** | **4** | **5** |
|---|---|---|---|---|---|
| Carbomer | 0,125 | 0,125 | 0,125 | 0,125 | 0,125 |
| Shea Butter | | | | 1,00 | |
| Mineralöl | | | | | 6,00 |
| Octyldodecanol | | | | 1,50 | |
| Caprylsäure/Caprinsäuretriglycerid | | | 4,00 | | |
| Dicaprylyl Carbonat | | 9,00 | | 1,00 | 3,00 |
| Dimethicon | | | | 0,50 | |
| Cyclomethicon | 9,00 | 2,00 | 3,00 | 2,00 | 1,00 |
| Diazolidinylharnstoff | 0,20 | 0,20 | 0,20 | 0,20 | 0,20 |
| Phenoxyethanol + Ethyl-, Methyl-, Propyl- Butyl-, Isobutylparaben | 0,50 | 0,50 | 0,50 | 0,50 | 0,50 |
| Parfum | | | | 0,25 | 0,25 |
| Glycerylstearatcitrat | 1,00 | 1,00 | 1,00 | 1,00 | 1,00 |
| Hydrierte Kokosfettsäureglycerid | 1,00 | 1,00 | 1,00 | 1,00 | 1,00 |
| Ammonium Acryloyldimethyltaurat/VP Copolymer | | | | | 0,125 |
| Hydroxyethylcellulose | 0,375 | 0,375 | 0,375 | 0,375 | 0,375 |
| Menthol | 0,10 | 0,50 | 1,00 | 0,10 | 0,10 |
| Wasser + Alcohol Denat | | | | 3,00 | |
| Wasser + Blue 1 | | 0,200 | 0,60 | 0,40 | |
| Wasser + Glycerin | 10,00 | 10,00 | 10,00 | 10,00 | 10,00 |
| Xanthangummi | 0,125 | 0,125 | 0,125 | 0,125 | |
| Wasser | ad 100,0 | ad 100,0 | ad 100,0 | ad 100,0 | ad 100,0 |
| Neutralisationsmittel (Natriumhydroxid, Kaliumhydroxid) | qs | qs | qs | qs | qs |
| pH-Wert | 4,5-5,5 | 6,5-8,5 | 5,0-7,0 | 4,0-6,0 | 5,0-7,5 |

### 17) Ölgele

| | **1** | **2** | **3** | **4** | **5** |
|---|---|---|---|---|---|
| Octyldodecanol | 9,00 | 9,00 | | | |
| Caprylsäure/Caprinsäuretriglycerid | 9,00 | | 6,00 | | |
| C12-15- Alkyl Benzoate | | | | 5,00 | 8,00 |
| Butylenglycol Dicaprylat/Dicaprat | | 9,00 | | | 8,00 |
| Dicaprylylether | 9,00 | | | 4,00 | |
| Dicaprylylcarbonat | | 7,00 | | | |
| Ethyl Galaktomannan (N-Hance® AG 200) | 3,50 | | | | 4,00 |
| C20-40 Fettsäuren + Polyethylen (Performacid®350) | | | | 3,60 | |
| Hydroxyoctacosanyl Hydroxystearat | 2,00 | | | | |
| Disteardimonium Hectorit | 1,00 | | | | 1,00 |
| Cetyl Dimethicon | 0,50 | | 4,50 | | |
| Cyclomethicon | | | 15,00 | | 5,00 |
| UVASorb® K2A | | | | | 1,00 |
| Uvinul® A Plus | 1,00 | 3,50 | 2,75 | 2,00 | 0,50 |
| Ethylhexylmethoxycinnamat | 6,00 | | | 10,00 | 3,0 |
| Octocrylen | 3,50 | | 7,50 | 10,00 | |
| Ethylhexylsalicylat | | 3,50 | | | 4,00 |
| Ethylhexyl Triazon | | | 2,00 | | |
| Diethylhexyl Butamido Triazon | | 0,50 | | 3,00 | 4,0 |
| Polysaccharid-N-alkylurethanen, Inulincarbamat | 1,50 | 4,50 | 5,50 | 1,00 | 3,00 |
| Phenoxyethanol | 0,50 | | | | |
| Parfüm, Farbstoff | q.s. | q.s. | q.s. | q.s. | q.s. |
| Mineralöl | ad. 100 | ad. 100 | ad. 100 | | |
| Reisöl | | | | ad. 100 | ad. 100 |

### 18) Feststoffstablisierte Emulsionen

| | **1** | **2** | **3** | **4** | **5** |
|---|---|---|---|---|---|
| Mineralöl | | | 16,00 | 16,00 | |
| Octyldodecanol | 9,00 | 9,00 | 5,00 | | |
| Caprylic/Capric Triglycerid | 9,00 | 9,00 | 6,00 | | |
| C12-15- Alkyl Benzoat | | | | 5,00 | 8,00 |
| Butylene Glykol Dicaprylat /Dicaprat | | | | | 8,00 |
| Dicaprylyl Ether | 9,00 | | | 4,00 | |
| Dicaprylyl Carbonat | | 9,00 | | | |
| Hydroxyoctacosanyl Hydroxystearat | 2,00 | 2,00 | 2,00 | 2,00 | 1,50 |
| Disteardimonium Hectorit | 1,00 | 0,750 | 0,50 | 0,50 | 0,25 |
| Cera Microcristallina + Paraffinöl | | | 2,50 | | 5,00 |
| Hydroxypropyl Methylcellulose | 0,15 | | | | 0,05 |
| Dimethicon | | | 4,50 | | |
| UVASorb® K2A | 2,00 | 5,00 | 3,00 | 1,50 | 1,00 |
| Bis-Ethylhexyloxyphenol Methoxypheny Triazin | 1,00 | 3,00 | 0,75 | 1,00 | 1,00 |
| Terephthaliden Dicampher Sulfonsäure | | 2,00 | | | 0,50 |
| Phenylbenzmidazol Sulfonsäure | 2,00 | 0,50 | 1,00 | | |
| Uvinul® A Plus | | | 2,75 | | 0,50 |
| Ethylhexylmethoxycinnamat | 6,00 | | | | 3,0 |
| Octocrylen | 3,50 | | 7,50 | | |
| Ethylhexyl Salicylat | | 3,50 | | | 4,00 |
| Diethylhexyl Butamido Triazon | | | | | 4,0 |
| Titandioxid Eusolex ® T-2000 | | 2,00 | 4,00 | 2,00 | 4,00 |
| Titandioxid T 805 ® | | | | | 3,00 |
| Silica Dimethyl Silylat | | | 1,00 | | |
| Bornitrid | 2,00 | | | 3,00 | |
| Tapioca Stärke | | | | 1,00 | |
| Natriumchlorid | 1,00 | 1,00 | 1,00 | 1,00 | |
| Glycerin | 5,0 | 10,0 | | 6,00 | 10,0 |
| Trinatrium EDTA | | 1,00 | | 1,00 | |
| Methylparaben | 0,21 | | | | 0,20 |
| Propylparaben | 0,07 | | | | |
| Phenoxyethanol | 0,50 | | 0,40 | 0,40 | 0,50 |
| Hexamidin Diisethionat | | | | | 0,08 |
| Diazolidinyl Harnstoff | | | 0,28 | 0,28 | |
| Alkohol | | 5,00 | | 2,50 | |
| Parfüm | 0,45 | 0,20 | | | 0,45 |
| Wasser | ad 100 | ad 100 | ad 100 | ad 100 | ad 100 |

### 19) Gele

### Eye Shadow Gel

| | Gew.-% |
|---|---|
| PEG-8 (Polyethylenglycol 400) | 2,00 |
| Ethanol | 5,00 |
| Aristoflex AVC | 1,50 |
| Glycerin | 2,00 |
| Panthenol | 0,50 |
| Tocopherolacetat | 0,50 |
| Timiron Splendid blue ® (Merck KgaA) | 4,50 |
| Chromoxid grün | 1,00 |
| Parfüm, Konservierungsmittel, NaOH, Komplexbildner, Farbstoffe, Antioxidantien etc. | q.s. |
| Wasser | ad 100,00 |

| **Highlighter Gel** | |
|---|---|
| | Gew. % |
| Carbomer | 1,50 |
| Glycerin | 2,50 |
| 1,3 Butylenglycol | 2,50 |
| Glitzerpigmente (z.B. Helicone HC Scarabeus, Wacker) | 1,00 |
| EDTA | 0,20 |
| Dimethicone | 1,50 |
| Parfüm, Konservierungsmittel, NaOH, | |
| Farbstoffe, Antioxidantien, etc. | q.s. |
| Wasser | ad 100,00 |

| **Eye Liner Gel** | |
|---|---|
| | Gew. % |
| Perlglanzpigmente | 10,00 |
| Eisenoxid | 3,00 |
| Silica | 2,00 |
| Aristoflex AVC | 1,00 |
| Hyroxyproylethyl Cellulose | 0,35 |
| Citric Acid | q.s. |
| Glycerin | 5,00 |
| PVP / VA Copolymer Parfüm, Konservierungsmittel, Farbstoffe, NaOH, | 2,00 |
| Komplexbildner, Antioxidantien, etc. | q.s. |
| Wasser | ad 100,00 |

### 20) Make-up

| **Emulsions-Make-up** | |
|---|---|
| | Gew.% |
| PEG-30 Stearat | 2,00 |
| Glycerinmonostearat | 1,00 |
| Stearinsäure | 1,00 |
| Cyclomethicon | 7,00 |
| Octyldodecanol | 7,00 |
| Isopropyl Lanolat | 4,00 |
| Squalan | 2,00 |
| Octyl Methoxycinnamat | 2,00 |
| Butyl Methoxydibenzoylmethan | 1,00 |
| Xanthan Gum | 0,20 |
| Glycerin | 5,00 |
| Butylenglkcol | 2,00 |
| Vitamin E Acetat | 1,00 |
| Magnesiumsilikat | 1,00 |
| Glimmer | 1,00 |
| Eisenoxid | 1,00 |
| Titandioxid | 2,50 |
| Talkum | 5,00 |
| EDTA | 0,50 |
| Parfüm, Konservierungsmittel, NaOH, Antioxidantien etc. | q.s. |
| Wasser | ad 100,00 |

### Emulsions-Make-Up

| | Gew. % |
|---|---|
| Cyclomethicon and PEG /PPG -18/18 Dimethicon | |
| (z.B. Dow Corning 3225 Formulation Aid) | 10,00 |
| Cyclomethicon | 10,00 |
| Bienenwachs | 3,00 |
| Polyglyceryl-4 Oleat | 2,00 |
| Eisenoxid | 1,00 |
| Titandioxid | 2,50 |
| Talkum | 12,00 |
| Natrium Chlorid | 2,00 |
| Parfüm, Konservierungsmittel, NaOH, Antioxidantien etc. | q.s. |
| Wasser | ad 100,00 |

| **Cover Cream** | |
|---|---|
| | Gew.% |
| Cyclomethicon | 44,00 |
| Bienenwachs | 3,00 |
| Carnaubawachs | 10,00 |
| Lanolinöl | 5,00 |
| Paraffinöl | 8,40 |
| Cetyl Alkohol | 2,60 |
| Eisenoxid | 3,00 |
| Titandioxid | 7,50 |
| Nylon | 6,00 |
| Talkum | 10,50 |
| Parfüm, Konservierungsmittel, Antioxidantien etc. | q.s. |

| **Emulsions-Make-up** | |
|---|---|
| | Gew. % |
| Cyclomethicon | 18,00 |
| Phenyltrimethicon | 3,00 |
| Cetyl PEG/PPG - 10/1 Dimethicon (z.B. Abil EM 90) | 4,00 |
| Paraffinöl | 3,00 |
| Eisenoxid | 2,30 |
| Titandioxid | 4,50 |
| Talkum | 2,00 |
| Natrium Chlorid | 2,00 |
| Quaternium-18 Hectorit | 0,30 |
| Propylen Carbonat | 0,08 |
| Parfüm, Konservierungsmittel, NaOH, Antioxidantien etc. | q.s. |
| Wasser | ad 100,00 |

## Patentansprüche

1. Kosmetisches Produkt umfassend einen Spender für pastöse Produkte mit einem das pastöse Produkt enthaltenden, im Wesentlichen zylindrischen Behälter (1), der bodenseitig einen unter dem Druck der Außenatmosphäre an einer Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, **dadurch gekennzeichnet dass** die Fördereinrichtung ein zu dem Behälter (1) und dem Kopfstück (3) längsverschiebliches Förderelement (5) umfasst, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlassöffnung (53) und eine Förderkanalauslassöffnung (58) aufweist, die durch eine Verschiebebewegung des Förderelementes (5) relativ zu dem Kopfstück (3) in eine Stellung bringbar ist, in der sich die Förderkanalauslassöffnung (58) zu dem Ausgabekanal (32) öffnet, sowie eine wasserhaltige kosmetisch und/oder dermatologische Zubereitung, die ein Hilfsmittel zur Gängighaltung des Spenders gewählt aus der Gruppe der Polyole mit 2 bis 6 Kohlenstoffatomen und 2 bis 6 Hydroxy- oder Alkoxygruppen und/oder der Tenside, die die Oberflächenspannung des Produktes auf <30mN/m herabsetzen.

2. Produkt nach Anspruch 1 **dadurch gekennzeichnet, dass** die Förderkanalauslassöffnung (58) an der Umfangsfläche des Förderschaftes (50) ausgespart ist und dass das Kopfstück (3) eine die Förderkanalauslassöffnung (58) in der Ausgangstellung (0) der Fördereinrichtung abdeckende Buchse aufweist.

3. Produkt nach Anspruch 1 oder 2 **dadurch gekennzeichnet, dass** die Buchse als eine die Fördereinrichtung längsverschieblich führende Führungsbuchse (31) ausgebildet ist, die wenigstens eine mit der Umfangsfläche des Förderschaftes (50) zusammenwirkende Führungsfläche aufweist.

4. Produkt nach einem der vorherigen Ansprüche 2 oder 3, **dadurch gekennzeichnet, dass** an dem Kopfstück (3) und der Fördereinrichtung Mitnehmermittel (34,57) vor gesehen sind, durch welche die Fördereinrichtung nach Handbetätigung bei Rückstellung des Kopfstücks (3) in die Ausgangsstellung (0) mitgenommen wird.

5. Produkt nach Anspruch 4, **dadurch gekennzeichnet, dass** an der Buchse (31) eine Mitnehmerschulter (57) ausgebildet ist, die mit einem an dem Förderschaft (50) an geformten Mitnehmerkranz (34) zusammenwirkt.

6. Produkt nach Anspruch 5, **dadurch gekennzeichnet, dass** die Mitnehmerschulter (34) endseitig an der Buchse (31) am Übergang zu dem Ausgabekanal (32) und der Mitnehmerkranz (57) im stirnseitigen Endbereich des Förderschaftes (50) vorgesehen sind.

7. Produkt nach einem der vorherigen Ansprüche, **dadurch gekennzeichnet, dass** der Förderkolben (51) den Förderschaft (50) zur Ausbildung einer ringförmigen Anlagefläche radial überragt und dass die Führungsbuchse (31) eine stirnseitige Druckfläche (33) aufweist, die in der Ausgangsstellung (0) mit axialem Abstand zu der Anlagefläche angeordnet und durch axiales Verschieben des Kopfstückes (3) in Richtung auf den Behälter (1) an die Anlagefläche anlegbar ist.

8. Produkt nach einem der vorherigen Ansprüche, **dadurch gekennzeichnet, dass** die Innenwand der Förderkammer (100) durch eine Innenhülse (13) gebildet ist, welche an der kopfstückseitigen Stirnseite des Behälters (1) an der dem Kopfstück (3) zu gewandten Seite an dem Behälter (1) vorgesehen ist.

9. Produkt nach einem der vorherigen Ansprüche, **gekennzeichnet durch** ein Kopfgegenstück (4), das einen topfförmig auf die Innenhülse (13) gestülpten Haltezylinder (41) sowie einen konzentrisch zu dem Haltezylinder (41) angeordneten, die Gleiterschiebung des Kopfstückes (3) führenden Führungszylinder (42) aufweist.

10. Produkt nach Anspruch 9, **dadurch gekennzeichnet, dass** das förderkammerseitige Ende des Führungszylinders (42) einen Förderkolbenanschlag für den Förderkolben (51) aufweist.

11. Produkt nach Anspruch 9 oder 10, **dadurch gekennzeichnet, dass** der Haltezylinder (41) mit einer bodenseitigen Ringschulter (44) versehen ist, die eine Anlagefläche für eine das Kopfstück in der Ausgangsstellung (0) unter Vorspannung haltende Schraubenfeder ausbildet und auf die Stirnseite des Behälters (1) aufgesetzt ist.

12. Produkt nach einem der Ansprüche 9 bis 11, **dadurch gekennzeichnet, dass** das Kopfgegenstück (4) wenigstens einen Anschlag (46a) zur Begrenzung der axialen Verschiebebewegung des Kopfstücks (3) aufweist und zusammen mit dem Kopfstück (3) als vorgefertigte Spenderkomponente ausgebildet und stirnseitig an dem Behälter (1) befestigt ist.

13. Produkt nach Anspruch 12, **dadurch gekennzeichnet, dass** die Spenderkomponente mit dem Behälter (1) über an dem Kopfgegenstück (4) und der Stirnseite des Behälters (1) ausgebildete Rastmittel (47; 17) verrastet ist.

14. Produkt nach einem der vorherigen Ansprüche, **dadurch gekennzeichnet, dass** das Kopfstück (3) derart längsverschieblich ist, dass das Kopfstück (3) mittels Handbetätigung von der Ausgangsstellung (0) zunächst um eine erste axiale Wegstrecke (a) zur Anlage an den Förderkolben bei gleichzeitiger Feilegung der Förderkanalauslassöffnung (58) in dem Ausgabekanal (32) in eine Mittelposition (M) bringbar ist und das Kopfstück (3) danach bei fortschreitender axialer Verschiebung unter Mitnahme des Förderkolbens (51) von der Mittelposition (M) in eine Ausgabe-Endposition (V) bringbar ist, in welcher die Förderkammer (100) durch Verschiebung des Förderkolbens (51) ihr kleinstes Volumen erreicht hat.

15. Produkt nach einem der vorherigen Ansprüche, **gekennzeichnet durch** ein an dem Kopfteil befestigtes Verschließteil (60), **durch** welches eine Produktabgabeöffnung (39) des Ausgabekanals (32) verschließbar ist.

16. Produkt nach Anspruch 15, **dadurch gekennzeichnet, dass** die Produktabgabeöffnung (39) ringförmig um einen in dem Ausgabekanal angeordneten Verschlussdorn (32a) ausgebildet ist und dass das Verschließteil (60) eine ringförmig ausgebildete, an den Verschlussdorn dichtend anlegbare Dichtlippe aufweist.

17. Produkt nach Anspruch 15 oder 16, **dadurch gekennzeichnet, dass** das Verschließteil (60) aus einer weichelastischen Kunststoffmasse, vorzugsweise aus einem thermoplastischen Elastomer gebildet ist.

18. Produkt nach einem der Ansprüche 15 bis 17, **dadurch gekennzeichnet, dass** das Verschließteil (60) einstückig mit einem zumindest stirnseitig an der Außenseite des Kopfteiles (3) ausgebildeten Überzug (61) ist.

19. Verwendung von Polyolen gewählt aus der Gruppe der Polyole mit 2 bis 6 Kohlenstoffatomen und 2 bis 6 Hydroxy- oder Alkoxygruppen, von Tensiden, die die Oberflächenspannung des Produktes auf <30mN/m herabsetzen in einem kosmetische und/oder dermatologische Zubereitungen enthaltenden Spender nach einem der Ansprüche 1 bis 18 als Hilfsmittel zur Gängighaltung des Spenders.

20. Produkt oder Verwendung nach einem der vorangehenden Ansprüche **dadurch gekennzeichnet, dass** das Polyol gewählt wird aus der Gruppe Glycerin, Buylenglycol, Propylenglycol, Dipropylenglycol, Pentandiol, Sorbitol, Ethylhexylglycerin und Panthenol. Bevorzugte Polyole sind Glycerin, Buylenglycol und Propylenglycol.

21. Produkt oder Verwendung nach einem der vorangehenden Ansprüche **dadurch gekennzeichnet, dass** der Gehalt an Polyol von mindestens 0,1 Gew.%, bevorzugt 0,5-20 Gew.%, besonders bevorzugt 1-10 Gew.%, bezogen auf das Gesamtgewicht der Zubereitung beträgt.

22. Produkt oder Verwendung nach einem der vorangehenden Ansprüche **dadurch gekennzeichnet, dass** ein Tensid mit einem HLB-Wert von mindestens 10 verwendet wird.

23. Produkt oder Verwendung nach einem der vorangehenden Ansprüche **dadurch gekennzeichnet, dass** als Tensid ein Alkylsulfat oder Alkylethersulfat gewählt wird.

24. Produkt oder Verwendung nach einem der vorangehenden Ansprüche **dadurch gekennzeichnet, dass** das Verhältnis aus Polyol zu Wasser 1:3 bis 1:25 beträgt.
